# EUROPEAN PATENT SPECIFICATION

(11) **EP 3 146 000 B1**
(45) Date of publication and mention of the grant of the patent: **26.06.2019**
(21) Application number: 15733565.4
(22) Date of filing: 20.05.2015
(51) Int. Cl.: C09D 4/00

(54) **COATING PROCESS**
BESCHICHTUNGSVERFAHREN
PROCÉDÉ DE REVÊTEMENT

(30) Priority: 22.05.2014 GB 201409095; 13.03.2015 GB 201504277
(43) Date of publication of application: 29.03.2017
(73) Proprietor: 3M Innovative Properties Company, St. Paul, MN 55133-3427 (US)
(72) Inventor: JINKS, Philip A., Bracknell Berkshire RG12 8HT (GB); AUDENAERT, Frans A., B-1831 Diegem (BE)
(74) Representative: Bergen, Katja
(86) International application number: PCT/US2015/031766
(87) International publication number: WO 2015/179511

(56) References cited:
- WO-A1-2010/129753
- WO-A2-2009/061891
- WO-A2-2009/061902
- WO-A2-2009/061907

## Description

The present invention relates to methods of making components for medicinal delivery devices, to components for medicinal delivery devices and to medicinal delivery devices. In particular, the present invention relates to methods of making components for medicinal inhalation devices, such components and such devices.

Medicinal delivery devices, including medicinal inhalation devices such as pressurized inhalers, e.g. metered dose pressurized inhalers (MDIs), and dry powder inhalers (DPIs), are widely used for delivering medicaments.

Medicinal delivery devices typically comprise a plurality of hardware components, (which in the case of a MDI can include gasket seals; metered dose valves (including their individual components, such as ferrules, valve bodies, valve stems, tanks, springs, retaining cups and seals); containers; and actuators) as well as a number of internal surfaces which may be in contact with the medicinal formulation during storage or come in contact with the medicinal formulation during delivery. In medicinal delivery devices, variability in dosing (i.e. from dose to dose) variability in dosing between units (i.e. device to device) is undesirable. Attention is paid to the design of medicinal delivery devices to reduce variability in particular by ensuring that dose volume, concentration and (if necessary) pressure are consistent dose to dose and unit to unit.

Often a desirable material for a particular component is found to be unsuitable in regard to its surface properties, e.g. surface energy, and/or its interaction with the medicinal formulation. Potentially undesirable interactions between a component and the medicinal formulation may include enhanced medicament degradation or permeation of a formulation constituent or extraction of chemicals from materials. For DPIs often permeation and adsorption of ambient water pose issues. Also the use of materials having relatively high surface energy for certain components (e.g. metered dose valves and/or individual components thereof), may have undesirable effects for the operation of movable components of a medicinal delivery device.

Various improvements have been proposed in for example, WO-A- 2005/026236, WO-A-2005/061572, WO-A-2002/30848, US-B-3 810 874, WO-A-2009/061891, WO-A-2009/061902, WO-A-2009/061907, WO-A-2010/129753 and WO-A-2010/129758.

Although a number of different improvements have been proposed, there is an ongoing need for medicinal delivery devices and components having desirable surface properties (e.g. low surface energy) as well as methods of providing such medicinal delivery devices and components.

The present invention accordingly provides, in a first aspect, a method of making a component for a medicinal delivery device, the method comprising
a) providing a component of a medicinal delivery device,
b) providing a primer composition comprising a silane having two or more reactive silane groups separated by an organic linker group,
c) providing a coating composition comprising an at least partially fluorinated compound,
d) applying the primer composition to at least a portion of the surface of the component,
e) applying the coating composition to the portion of the surface of the component after application of the primer composition.

Previously, it was thought that aspects relating to the design or structure of medicinal delivery devices and not surface properties of medicinal delivery devices were the most important contributors to dose to dose and unit to unit variability, for example in e.g. MDI, differences in dose to dose delivery volume or concentration and unit to unit tolerances in formulation, pressure, or volume.

However, surprisingly, the inventors have discovered that methods according to the present invention provide components which, when assembled and in use, significantly reduce dose to dose variability of medicinal delivery devices. Additionally, such methods provide components which, when assembled and in use, provide reduced unit to unit variability.

The at least partially fluorinated compound will usually comprise one or more reactive functional groups, with the or each one reactive functional group usually being a reactive silane group, for example a hydrolysable silane group or a hydroxysilane group. Such reactive silane groups allow reaction of the partially fluorinated compound with one or more of the reactive silane groups of the primer. Often such reaction will be a condensation reaction.

The reactive silane group may be of formula - Si(R⁰)ₙX₃₋ₙ, wherein R⁰ is a substantially non-hydrolysable group, X is a hydrolysable or hydroxy group and n is 0, 1 or 2.

Thus, in many embodiments the silane having two or more reactive silane groups is of formula

X₃₋ₘ(R¹)ₘSi-Q-Si(R²)ₖX₃₋ₖ

wherein R¹ and R² are independently selected univalent groups such as C1-C4 alkyl, X is a hydrolysable or hydroxy group, m and k are independently 0, 1, or 2 and Q is a divalent organic linking group.

Usually, Q will comprise a 1 to 12 atom chain, more usually a substituted or unsubstituted C₂ to C₁₂ hydrocarbyl chain. Preferably, Q comprises a substituted or unsubstituted C₂ to C₁₂ alkyl chain.

Useful examples of silanes having two or more reactive silane groups include one or a mixture of two or more of 1,2- bis(trialkoxysilyl) ethane, 1,6- bis(trialkoxysilyl) hexane, 1,8- bis(trialkoxysilyl) octane, 1,4-bis(trialkoxysilylethyl)benzene, bis(trialkoxysilyl)itaconate, and 4,4'-bis(trialkoxysilyl)-1,1'-diphenyl, wherein any trialkoxy group may be independently trimethoxy or triethoxy.

Q may comprise a chain substituted by one or more atoms of N, O, and/or S. Thus, further examples of the silane having two or more reactive silane groups include one or a mixture of two or more of bis(trialkoxysilylpropyl)amine; bis (3-trialkoxysilylpropyl) ethylenediamine; bis (3-trialkoxysilylpropyl) n-methylamine; bis[3-(trialkoxysilyl)propyl]fumarate and N, N-bis (3-trialkoxysilylmethyl) allylamine, wherein any trialkoxy group may be independently trimethoxy or triethoxy.

In preferred embodiments, the silane is such that Q may be of formula - (CH₂)ᵢ -A-(CH₂)ⱼ - wherein A is NRⁿ, O, or S; i and j are independently 0, 1, 2, 3 or 4 and wherein Rⁿ is H or C₁ to C₄ alkyl. Even more preferably, Q may be of formula - (CH₂)ᵢ -NH - (CH₂)ⱼ - and i and j are each independently 1, 2, 3 or 4. Most preferably i and j are each 3.

The coating solvent usually comprises an alcohol or a hydrofluoroether.

If the coating solvent is an alcohol, preferred alcohols are C₁ to C₄ alcohols, in particular, an alcohol selected from ethanol, n-propanol, or iso-propanol or a mixture of two or more of these alcohols.

If the coating solvent is an hydrofluoroether, it is preferred if the coating solvent comprises a C₄ to C₁₀ hydrofluoroether. Generally, the hydrofluoroether will be of formula

C_{g}F_{2g+1}OCₕH₂ₕ₊₁

wherein g is 2, 3, 4, 5, or 6 and h is 1, 2, 3 or 4. Examples of suitable hydrofluoroethers include those selected from the group consisting of methyl heptafluoropropylether, ethyl heptafluoropropylether, methyl nonafluorobutylether, ethyl nonafluorobutylether and mixtures thereof.

The at least partially fluorinated compound may contain a polyfluoroether moiety, in particular a polyfluoropolyether moiety. More particularly, the polyfluoroether moiety may be a perfluorinated polyfluoroether moiety, even more particularly the polyfluoroether moiety may be a perfluorinated polyfluoropolyether moiety.

The polyfluoropolyether silane may be of formula

R*^{f}*Q¹ᵥ[Q²_{w}-[C(R⁴)₂-Si(X)₃₋ₓ(R⁵)ₓ]_{y}]_{z}

wherein:
R*^{f}* is a polyfluoropolyether moiety;
Q¹ is a trivalent linking group;
each Q² is an independently selected organic divalent or trivalent linking group;
each R⁴ is independently hydrogen or a C₁₋₄ alkyl group;
each X is independently a hydrolysable or hydroxyl group;
R⁵ is a C₁₋₈ alkyl or phenyl group;
v and w are independently 0 or 1, x is 0 or 1 or 2; y is 1 or 2; and z is 2, 3, or 4.

The polyfluoropolyether moiety R*^{f}* may comprise perfluorinated repeating units selected from the group consisting of -(CₙF₂ₙO)-,-(CF(Z)O)-, (CF(Z)CₙF₂ₙO)-, - (CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6. In particular, n may be an integer from 1 to 4, more particularly from 1 to 3. For repeating units including Z the number of carbon atoms in sequence may be at most four, more particularly at most 3. Usually, n is 1 or 2 and Z is an -CF₃ group, more wherein z is 2, and R*^{f}* is selected from the group consisting of -CF₂O(CF₂O)ₘ(C₂F₄O)ₚCF₂-, -CF(CF₃)O(CF(CF₃)CF₂O)ₚCF(CF₃)-, -CF₂O(C₂F₄O)ₚCF₂-, -(CF₂)₃O(C₄F₈O)ₚ(CF₂)₃-, -CF(CF₃)-(OCF₂CF(CF₃))ₚO-CₜF₂ₜ-O(CF(CF₃)CF₂O)ₚCF(CF₃)-, wherein t is 2, 3 or 4 and wherein m is 1 to 50, and p is 3 to 40.

The composition may further comprise a cross-linking agent. The cross-linking agent may comprise a compound selected from group consisting of tetramethoxysilane; tetraethoxysilane; tetrapropoxysilane; tetrabutoxysilane; methyl triethoxysilane; dimethyldiethoxysilane; octadecyltriethoxysilane; 3-glycidoxy-propyltrimethoxysilane; 3-glycidoxy-propyltriethoxysilane; 3-aminopropyl-trimethoxysilane; 3-aminopropyl-triethoxysilane; bis(3-trimethoxysilylpropyl) amine; 3-aminopropyl tri(methoxyethoxyethoxy) silane; N(2-aminoethyl)3-aminopropyltrimethoxysilane; bis( 3-trimethoxysilylpropyl) ethylenediamine; 3-mercaptopropyltrimethoxysilane; 3-mercaptopropyltriethoxysilane; 3-trimethoxysilyl-propylmethacrylate; 3-triethoxysilypropylmethacrylate; bis(trimethoxysilyl) itaconate; allyltriethoxysilane; allyltrimethoxysilane; 3-(N-allylamino)propyltrimethoxysilane; vinyltrimethoxysilane; vinyltriethoxysilane; and mixtures thereof.

Usually, the method may include a pre-treatment step prior to the step of applying the primer composition, said pre-treatment step in particular may comprise cleaning the surface with a solvent comprising a hydrofluoroether, e.g. HFE72DE an azeotropic mixture of about 70%w/w trans-dichloroethylene; 30% w/w of a mixture of methyl and ethyl nonafluorobutyl and nonafluoroisobutyl ethers.

The hydrofluoroether for the cleaning step may be selected from the group consisting of methyl heptafluoropropylether; ethyl heptafluoropropylether; methyl nonafluorobutylether; ethyl nonafluorobutylether and mixtures thereof.

In the method, the primer composition and/or the coating composition may be, independently of the application step used for the other, applied by spraying, dipping, rolling, brushing, spreading or flow coating, in particular by spraying or dipping.

In the method, after the step of applying either the primer composition or after the step of applying the coating composition, the method may further comprise a step of curing. The curing step may be carried out at an elevated temperature in the range from about 40°C to about 300°C.

The primer may be cured by evaporating the solvent in a moist environment, as water is responsible for the curing, and may be aided by heat or elevated humidity.

If heat is applied to the primer coating, it is preferable to allow the prime-coated component to cool to room temperature before applying the top coating.

Typically, a lower curing temperature is used than for coating polymers than for coating metals, to avoid deformation of the polymer. However, it has been found that it is not essential to ensure that the primer is cured before applying the top coating, as a final curing can be effectively done to both layers simultaneously, thereby simplifying the manufacturing process. Hence, the primer coat may be allowed to set on the surface such that it is not washed off by the subsequent top coating, by allowing (e.g. by leaving to stand) or causing (e.g. by blowing in a current of air) most of the primer solution solvent to be dispersed.

Coatings applied to valve components, such as plastic stems, metal stems or elastomeric seals, in which the at least partially fluorinated compound is a perfluoropolyether silane according to formula Ia in which R^{f} comprises from 20 to 40 linked repeating units confer additional lubricity compared to those with fewer repeating units, and when these are assembled with other component to make up valves, the valves have lower actuation forces.

Formula Ia is :

R^{f}[Q¹-[C(R)₂-Si(Y)₃₋ₓ(R^{1a})ₓ]_{y}]_{z} Ia

wherein:
R*^{f}* is a monovalent or multivalent polyfluoropolyether moiety;
Q¹ is an organic divalent or trivalent linking group;
each R is independently hydrogen or a C1-4 alkyl group;
each Y is independently a hydrolysable group;
R^{1a} is a C1-8 alkyl or phenyl group;
x is 0 or 1 or 2;
y is 1 or 2; and
z is 1, 2, 3, or 4.

The surface may be a metal surface, in particular a surface of an aluminium alloy, an iron alloy, or a steel alloy.

The surface may be a polymer surface. The polymer may be a thermoplastic or thermoset material.

The types of plastics that can thus be coated includes polyalkylenes, polyesters, polyoxymethylene, poly(acrylonitrile-butadiene-styrene) (ABS) or other copolymers comprising monomers of acrylonitrile, butadiene and styrene, such as methylmethacrylate-acrylonitrile-butadiene-styrene (MABS), polycarbonate and nylon, e.g. nylon 6,6 or nylon 6,12. Examples of polyalkylenes are polypropylene and polyethylenes such as low density polyethylene (LDPE), ultra-high molecular weight polyethylene (UHMPE). Polyesters include polyethylene terephthalate (PET) and polybutylene terephthalate (PBT) and copolyester.

Thermosets that may be usefully coated include nitrile elastomer, neoprene elastomer, EPDM and co-vulcanisates of elastomeric polymers with thermoplastic polymers.

Medicinal delivery devices may include inhalers, or other dispensers in which accurate dosing depends on not leaving medicament behind on the device. Examples include a pressurized metered dose inhaler (pMDI), a nebulizer and a dry powder inhaler (DPI). When the device is a pMDI, examples of components include an actuator, an aerosol container, a ferrule, a valve body, a valve stem and a compression spring. For DPIs, examples of components include a powder container, a component used to open sealed powder container, a component that defines at least in part a deagglomeration chamber, a component of a deagglomeration system, a component that defines at least in part a flow channel, a dose-transporting component, a component that defines at least in part a mixing chamber, a component that defines at least in part an actuation chamber, a mouthpiece and a nosepiece.

Components to be coated may be made from one of the above materials, or from assemblies or co mouldings of more than one material including at least one of the above material types. Alternatively, plastics coatings on components may provide a surface amenable to further coating.

It has been found that the deposition of drug on plastics or thermoset components can be reduced when the component is coated by applying a primer coat of a silane having two or more reactive silane groups separated by an organic linker, followed by a top coating of an at least partially fluorinated compound.

The primer preferably comprises a linking group with a chain of one to twelve atoms. This may have at least one carbon atom either side of a heteroatom, such as nitrogen, oxygen or sulphur. Particularly useful primers include at least one amine group in the linking group.

It has surprisingly been found that deposition of drug on medicinal delivery device component surfaces is eliminated when the organic linker comprises one or more amine groups and the at least partially fluorinated compound is a perfluoropolyether silane according to formula Ia in which y=z=1.

The surface of the device or the surface of the component of the device, as applicable, is preferably a surface that is or is intended to come in contact with a medicament or a medicinal formulation during storage or delivery from the medicinal delivery device.

The medicament may comprise or the medicinal formulation may comprise a medicament that may be a drug, vaccine, DNA fragment, hormone or other treatment. Suitable drugs include those for the treatment of respiratory disorders, e.g., bronchodilators, anti-inflammatories (e.g., corticosteroids), anti-allergics, anti-asthmatics, anti-histamines, and anti-cholinergic agents. Therapeutic proteins and peptides may also be employed for delivery by inhalation. Thus the medicament may be selected from the an exemplary group consisting of albuterol, terbutaline, ipratropium, oxitropium, tiotropium, TD-4208, beclomethasone, flunisolide, budesonide, mometasone, ciclesonide, cromolyn sodium, nedocromil sodium, ketotifen, azelastine, ergotamine, cyclosporine, aclidinium, umeclidinium, glycopyrrolate, salmeterol, fluticasone, formoterol, procaterol, indacaterol, TA2005, milveterol, olodaterol, vilanterol, abediterol, omalizumab, zileuton, insulin, pentamidine, calcitonin, leuprolide, alpha-1-antitrypsin, interferon, triamcinolone, salbutamol, pharmaceutically acceptable salts and esters of any of the listed medicaments and mixtures of any of the listed medicaments, their pharmaceutically acceptable salts and esters. For fluticasone, the preferred esters are propionate or furoate; for mometasone, the preferred ester is furoate.

The medicinal delivery device is preferably a metered dose inhaler or a dry powder inhaler. Thus, preferably, the component (preferably comprising metal) is a component of a metered dose inhaler and the component is selected from the group consisting of an actuator, an aerosol container, a ferrule, a valve body (that defines a metering chamber), a bottle emptier, a valve stem and a compression spring.

An aerosol formulation used in a metered dose inhaler typically comprises a medicament or a combination of medicaments (as discussed above) and liquefied propellant selected from the group consisting of HFA 134a, HFA 227 and mixtures thereof.

Aerosol formulations may, as desired or needed, comprise other excipients, such as surfactant, a co-solvent (e.g., ethanol), CO₂, or a particulate bulking agent. Medicament may be provided in particulate form (e.g. particles generally having a median diameter in the range of 1 to 10 microns) suspended (i.e. dispersed) in the liquefied propellant. Alternatively medicament may be in solution (i.e. dissolved) in the formulation. In the event a combination of two or more medicaments is used, all the medicaments may be suspended or in solution or alternatively one or more medicaments may be suspended, while one or more medicaments may be in solution.

The amount of medicament may be determined by the required dose per puff and available valve sizes, which for MDIs are typically 25, 50 or 63 microlitres, or 100 microlitres..

Pressurized metered dose inhalers including e.g., aerosol containers (in particular metal aerosol containers) whose interior surfaces are coated in accordance the invention are particularly advantageous for containing and delivering medicinal aerosol formulations comprising a medicament that is dispersed in said formulation.

In addition embodiments in accordance with the present invention are particularly useful in regard to metered dose inhalers including a medicinal aerosol formulation that includes low amounts of surfactant (0.005 wt % with respect to the formulation); or is substantially free (less than 0.0001 wt % with respect to drug) or free of a surfactant. Alternatively or additionally, embodiments, are particularly useful in metered dose inhalers including a medicinal aerosol formulation that contains low amounts of ethanol (less than 5 wt % with respect to the formulation), or is substantially free (less than 0.1 wt % with respect to the formulation) or free of ethanol.

In a second aspect, the present invention provides a coated component for a medicinal delivery device comprising a component and a fluorine-containing coating, wherein the fluorine-containing coating comprises two layers, a first polyfluoropolyether-containing layer comprising polyfluoropolyether silane entities of the following Formula Ib:

R*^{f}*[Q¹-[C(R)₂-Si(O-)₃₋ₓ(R^{1a})ₓ]_{y}]_{z} Ib

which shares at least one covalent bond with a second non-fluorinated layer comprising entities of the following Formula IIb:

(-O)₃₋ₘ₋ₙ(X)ₙ(R¹)ₘSi-Q-Si(R²)ₖ(X)ₗ(O-)₃₋ₖ₋ₗ IIb

which in turn shares at least one covalent bond with the component; and wherein:
R*^{f}* is a monovalent or multivalent polyfluoropolyether segment;
Q¹ is an organic divalent or trivalent linking group;
each R is independently hydrogen or a C1-4 alkyl group;
R^{1a} is a C1-8 alkyl or phenyl group;
k, l, m and n are independently 0, 1 or 2, but with the priviso that m+n and k+l are at most 2;
x is 0 or 1 or 2; y is 1 or 2; and z is 1, 2, 3, or 4; wherein R¹ and R² are independently selected univalent groups, X is a hydrolysable or hydroxy group, m and k are independently 0, 1, or 2 and Q is a divalent organic linking group, comprising a substituted C₂ to C₁₂ hydrocarbyl chain and one or more amine groups.
   Coated components with more specific surface structural features may be determined by the skilled person in respect of more specific process steps described earlier by taking into account the covalent bonding chemistry of this third aspect of the invention.

The invention, in its various combinations, either in method or apparatus form, may also be characterized by the following listing of items:

### Items

1. A method of making a component for a medicinal delivery device, the method comprising
   a) providing a component of a medicinal delivery device,
   b) providing a primer composition comprising a silane having two or more reactive silane groups separated by an organic linker group,
   c) providing a coating composition comprising an at least partially fluorinated compound,
   d) applying the primer composition to at least a portion of the surface of the component,
   e) applying the coating composition to the portion of the surface of the component after application of the primer composition.
2. A method as referred to in item 1, wherein the at least partially fluorinated compound comprises one or more reactive silane groups.
3. A method as referred to in either item 1or item 2, wherein at least one of the reactive silane groups is a hydrolysable silane group or a hydroxysilane group.
4. A method as referred to in item 3, wherein the or each reactive silane group is of formula - Si(R⁰)ₙX₃₋ₙ, wherein R⁰ is a substantially non-hydrolysable group, X is a hydrolysable or hydroxy group and n is 0, 1 or 2.
5. A method as referred to in any one of the preceding items, wherein the silane having two or more reactive silane groups is of formula

   X₃₋ₘ(R¹)ₘSi-Q-Si(R²)ₖX₃₋ₖ

   wherein R¹ and R² are independently selected univalent groups, X is a hydrolysable or hydroxy group, m and k are independently 0, 1, or 2 and Q is a divalent organic linking group.
6. A method as referred to in item 5, wherein Q comprises a 1 to 12 atom chain.
7. A method as referred to in item 6, wherein Q comprises a substituted or unsubstituted C₂ to C₁₂ hydrocarbyl chain.
8. A method as referred to in item 7, wherein the silane having two or more reactive silane groups is selected from 1,2-bis(trialkoxysilyl) ethane, 1,6-bis(trialkoxysilyl) hexane, 1,8-bis(trialkoxysilyl) octane, 1,4-bis(trialkoxysilylethyl)benzene, bis(trialkoxysilyl)itaconate, and 4,4'-bis(trialkoxysilyl)-1,1'-diphenyl, and combinations thereof, wherein any trialkoxy group may be trimethoxy or triethoxy.
9. A method as referred to in item 7, wherein Q comprises a substituted or unsubstituted C₂ to C₁₂ alkyl chain.
10. A method as referred to in either item 7 or item 8, wherein the chain is substituted by one or more atoms of N, O, and/or S.
11. A method as referred to in item 9, wherein the silane having two or more reactive silane groups is selected from bis(trialkoxysilylpropyl)amine; bis(3-trialkoxysilylpropyl) ethylenediamine; bis(3-trialkoxysilylpropyl)n-methylamine; bis[3-(trialkoxysilyl)propyl]fumarate and N, N-bis(3-trialkoxysilylmethyl) allylamine, and combinations thereof, wherein any trialkoxy group may be trimethoxy or triethoxy.
12. A method as referred to in item 10, wherein Q is of formula - (CH₂)ᵢ-A-(CH₂)ⱼ - wherein A is NRⁿ, O, or S; i and j are independently 0, 1, 2, 3 or 4 and wherein Rⁿ is H or C₁ to C₄ alkyl.
13. A method as referred to in item 12, wherein Q is of formula - (CH₂)ᵢNH(CH₂)ⱼ - and i and j are each independently 1, 2, 3 or 4.
14. A method as referred to in any one of items 4 to 13, wherein each X is OR³, each R³ being independently hydrogen, phenyl or C₁ to C₄ alkyl.
15. A method as referred to in item 14, wherein each R³ is independently methyl, ethyl or propyl.
16. A method as referred to in any one of the preceding items, wherein the coating composition further comprises a coating solvent.
17. A method as referred to in item 16, wherein the coating solvent comprises an alcohol or a hydrofluoroether.
18. A method as referred to in item 17, wherein the alcohol is a C₁ to C₄ alcohol, in particular, an alcohol selected from ethanol, n-propanol, or iso-propanol.
19. A method as referred to in either item 16 or item 17, wherein the hydrofluoroether is a C₄ to C₁₀ hydrofluoroether.
20. A method as referred to in item 19, wherein the hydrofluoroether is of formula

   C_{g}F_{2g+1}OCₕH₂ₕ₊₁

   wherein g is 2, 3, 4, 5, or 6 and h is 1, 2, 3 or 4.
21. A method as referred to in item 20, wherein the hydrofluoroether is selected from the group consisting of methyl heptafluoropropylether, methyl nonafluorobutylether, ethyl nonafluorobutylether and mixtures thereof.
22. A method as referred to in item 20, wherein the hydrofluoroether comprises ethyl nonafluorobutyl ether.
23. A method as referred to in any one of the preceding items wherein the at least partially fluorinated compound contains a polyfluoroether moiety, in particular a polyfluoropolyether moiety.
24. A method as referred to in item 23, wherein the polyfluoroether moiety is a perfluorinated polyfluoroether moiety, in particular a perfluorinated polyfluoropolyether moiety.
25. A method as referred to in either item 23 or item 24, wherein the polyfluoropolyether moiety is not linked to the functional silane groups via a functionality that includes nitrogen-silicon bond or a sulfur-silicon bond.
26. A method as referred to in any one of items 23 to 25, wherein the polyfluoropolyether moiety is linked to the functional silane groups via a functionality that includes a carbon-silicon bond.
27. A method as referred to in any one of items 23 to 26, wherein in the repeating units of the polyfluoropolyether moiety, the number of carbon atoms in sequence is at most 6.
28. A method as referred to in item 27, wherein in the repeating units of the polyfluoropolyether moiety the number of carbon atoms in sequence is 4 or fewer, more particularly 3 or fewer.
29. A method as referred to in any one of items 23 to 28, wherein the polyfluoropolyether silane is of formula

   R*^{f}*Q¹ᵥ[Q²_{w}-[C(R⁴)₂-Si(X)₃₋ₓ(R⁵)ₓ]_{y}]_{z}

   wherein:
   R*^{f}* is a polyfluoropolyether moiety;
   Q¹ is a trivalent linking group;
   each Q² is an independently selected organic divalent or trivalent linking group;
   each R⁴ is independently hydrogen or a C₁₋₄ alkyl group;
   each X is independently a hydrolysable or hydroxyl group;
   R⁵ is a C₁₋₈ alkyl or phenyl group;
   v and w are independently 0 or 1, x is 0 or 1 or 2; y is 1 or 2; and z is 2, 3, or 4.
30. A method as referred to in item 29, wherein the polyfluoropolyether moiety R*^{f}* comprises perfluorinated repeating units selected from the group consisting of -(CₙF₂ₙO)-,-(CF(Z)O)-, -(CF(Z)CₙF₂ₙO)-,-(CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6.
31. A method as referred to in item 30, wherein n is an integer from 1 to 4 and wherein for repeating units including Z the number of carbon atoms in sequence is at most four.
32. A method as referred to in either item 30 or item 31, wherein n is an integer from 1 to 3 and wherein for repeating units including Z the number of carbon atoms in sequence is at most three.
33. A method as referred to in any one of items 30 to 32, wherein n is 1 or 2 and Z is an -CF₃ group.
34. A method as referred to in any one of items 29 to 33, wherein z is 2, and R*^{f}* is selected from the group consisting of -CF₂O(CF₂O)ₘ(C₂F₄O)ₚCF₂-, -CF(CF₃)O(CF(CF₃)CF₂O)ₚCF(CF₃)-, -CF₂O(C₂F₄O)ₚCF₂-, -(CF₂)₃O(C₄F₈O)ₚ(CF₂)₃-, -CF(CF₃)-(OCF₂CF(CF₃))ₚO-CₜF₂ₜ-O(CF(CF₃)CF₂O)ₚCF(CF₃)-, wherein t is 2, 3 or 4 and wherein m is 1 to 50, and p is 3 to 40.
35. A method as referred to in item 34, wherein R*^{f}* is selected from the group consisting of -CF₂O(CF₂O)ₘ(C₂F₄O)ₚCF₂-, -CF₂O(C₂F₄O)ₚCF₂-, and -CF(CF₃)-(OCF₂CF(CF₃))ₚO-(CₜF₂ₜ)-O(CF(CF₃)CF₂O)ₚCF(CF₃)-, and wherein t is 2, 3, or 4, and wherein the average value of m+p or p+p or p is from about 4 to about 24.
36. A method as referred to in any one of items 29 to 35, wherein Q¹and Q² are independently selected from the group consisting of -C(O)N(R³)-(CH₂)ₐ-, -S(O)₂N(R³)-(CH₂)ₐ-, -(CH₂)ₐ-, -CH₂O-(CH₂)ₐ-, -C(O)S-(CH₂)ₐ-, -CH₂OC(O)N(R³)-(CH₂)ₐ-, and wherein R³ is hydrogen or C₁₋₄alkyl, and a is 1 to about 25.
37. A method as referred to in any one of items 23 to 36, wherein the weight average molecular weight of the polyfluoropolyether moiety is about 1000 or higher, in particular about 1800 or higher.
38. A method as referred to in any one of the preceding items, wherein the composition further comprises a cross-linking agent.
39. A method as referred to in item 38, wherein the cross-linking agent comprises a compound selected from the group consisting of tetramethoxysilane; tetraethoxysilane; tetrapropoxysilane; tetrabutoxysilane; methyl triethoxysilane; dimethyldiethoxysilane; octadecyltriethoxysilane; 3-glycidoxy-propyltrimethoxysilane; 3-glycidoxy-propyltriethoxysilane; 3-aminopropyl-trimethoxysilane; 3-aminopropyl-triethoxysilane; bis(3-trimethoxysilylpropyl) amine; 3-aminopropyl tri(methoxyethoxyethoxy) silane; N(2-aminoethyl)3-aminopropyltrimethoxysilane; bis( 3-trimethoxysilylpropyl) ethylenediamine; 3-mercaptopropyltrimethoxysilane; 3-mercaptopropyltriethoxysilane; 3-trimethoxysilyl-propylmethacrylate; 3-triethoxysilypropylmethacrylate; bis(trimethoxysilyl) itaconate; allyltriethoxysilane; allyltrimethoxysilane; 3-(N-allylamino)propyltrimethoxysilane; vinyltrimethoxysilane; vinyltriethoxysilane; and mixtures thereof.
40. A method as referred to in any one of the preceding items, wherein the method includes a pre-treatment step prior to the step of applying the primer composition, said pre-treatment step comprising cleaning the surface with a solvent comprising a hydrofluoroether.
43. A method as referred to in any one of the preceding items, wherein said surface is a metal surface, in particular a surface of an aluminium alloy, an iron alloy, or a steel alloy.
44. A method as referred to in any one of the preceding items, wherein independently the primer composition and/or the coating composition are applied by spraying, dipping, rolling, brushing, spreading or flow coating, in particular by spraying or dipping.
45. A method as referred to in any one of the preceding items, wherein after applying the composition, the method further comprises a step of curing.
46. A method as referred to in item 45, wherein the curing is carried out at an elevated temperature in the range from about 40°C to about 300°C.
47. A method as referred to in any one of the preceding items, where said surface of the device or said surface of the component of the device, as applicable, is a surface that is or will come in contact with a medicament or a medicinal formulation during storage or delivery from the medicinal delivery device.
48. A method as referred to in item 47, wherein the medicament comprises or the medicinal formulation comprises a medicament selected from the group consisting of albuterol, terbutaline, ipratropium, oxitropium, tiotropium, beclomethasone, flunisolide, budesonide, mometasone, ciclesonide, cromolyn sodium, nedocromil sodium, ketotifen, azelastine, ergotamine, cyclosporine, salmeterol, fluticasone, formoterol, procaterol, indacaterol, TA2005, omalizumab, zileuton, insulin, pentamidine, calcitonin, leuprolide, alpha-1-antitrypsin, interferon, triamcinolone, salbutamol and pharmaceutically acceptable salts and esters thereof and mixtures thereof.
49. A method as referred to in any one of the preceding items, where said medicinal delivery device is a metered dose inhaler or a dry powder inhaler.
50. A method as referred to in any one of the preceding items, wherein the component is a component of a metered dose inhaler and the component is selected from the group consisting of an actuator, an aerosol container, a ferrule, a valve body, a valve stem and a compression spring.
51. A medicinal delivery device assembled from at least one component made as referred to in any one of the preceding items.
52. A method as referred in any one of items 30 to 50, wherein the number of linked perfluorinated repeating units is in the range 20 to 40.
53. A method as referred to in any one of items 1 to 39, 44 to 50 and 52, wherein said portion of surface is a polymer surface.
54. A method as referred to in item 53 wherein the component is at least partly made of said polymer.
55. A method as referred to in any one of items 53 and 54, wherein the silane having two or more reactive silane groups is of formula

   X₃₋ₘ(R¹)ₘSi-Q-Si(R²)ₖX₃₋ₖ

   wherein R¹ and R² are independently selected univalent groups, X is a hydrolysable or hydroxy group, m and k are independently 0, 1, or 2 and Q is a divalent organic linking group, comprising a substituted C₂ to C₁₂ hydrocarbyl chain and one or more amine groups.
56. A method as referred to in any one of items 53 to 55, wherein the at least partially fluorinated compound is polyfluoropolyether silane of the Formula Ia:

   R*^{f}*[Q¹-[C(R)₂-Si(Y)₃₋ₓ(R^{1a})ₓ]_{y}]_{z} Ia

   wherein:
   R*^{f}* is a monovalent or multivalent polyfluoropolyether moiety;
   Q¹ is an organic divalent or trivalent linking group;
   each R is independently hydrogen or a C1-4 alkyl group;
   each Y is independently a hydrolysable group;
   R^{1a} is a C1-8 alkyl or phenyl group;
   x is 0 or 1 or 2;
   y is 1 or 2; and
   z is 1, 2, 3, or 4.
57. A method as referred to in item 56, wherein the polyfluoropolyether moiety R*^{f}* comprises perfluorinated repeating units selected from the group consisting of -(CₙF₂ₙO)-,-(CF(Z)O)-, -(CF(Z)CₙF₂ₙO)-, -(CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6.
58. A method as referred to in item 57, wherein the number of linked perfluorinated repeating units is in the range 3 to 50.
59. A method as referred to in item 58, wherein the number of linked perfluorinated repeating units is in the range 20 to 40.
60. A method as referred to in any one of items 56 to 59, wherein the polyfluoropolyether moiety R*^{f}* is terminated with a group selected from the group consisting of CₙF₂ₙ₊₁-, CₙF₂ₙ₊₁O-, HCₙF₂ₙO-.
61. A method as referred to in item 60, wherein n=1,2,3,4,5 or 6.
62. A method as referred to in item 61, wherein the average structure of the polyfluoropolyether moiety R*^{f}* is selected from: C₃F₇O(CF(CF₃)CF_{2O})ₚCF(CF₃)-, CF₃O(C₂F₄O)ₚCF₂-, C₃F₇O(CF(CF₃)CF₂O)ₚCF₂CF₂-,C₃F₇O(CF₂CF₂CF₂O)ₚCF₂CF₂-, or C₃F₇O(CF₂ CF₂CF₂O)ₚCF(CF₃)-, or CF₃O(CF₂CF(CF₃)O)ₚ(CF₂O)G- (wherein G is CF₂, C₂F₄-, C₃F6- or C₄F₈-), and wherein the average value of p is in the range 3 to 50.
63. A method as referred to in item 62, wherein the polyfluoropolyether moiety R*^{f}* is C₃F₇O(CF(CF₃)CF₂O)ₚCF(CF₃)-
64. A method as referred to in any one of items 56to 63, wherein z=1.
65. A method as referred to in any one of items 56 to 64, wherein y=1.
66. A method as referred to in any one of items 56 to 65, wherein Q¹ contains one or more heteroatoms selected from the group consisting of sulfur, oxygen, and nitrogen.
67. A method as referred to in item 66 wherein Q¹ contains one or more functional groups selected from the group consisting of esters, amides, sulfonamides, carbonyl, carbonates,
   ureylenes, and carbamates.
68. A method as referred to in item 67 wherein Q¹ comprises from 2 to 25 linearly arranged carbon atoms, optionally interrupted by one or more heteroatoms.
69. A method as referred to in item 68 wherein Q¹ is substantially stable against hydrolysis and other chemical transformations, such as nucleophilic attack.
70. A method as referred to in item 69 wherein Q¹ includes one or more organic linking groups selected from:- -C(O)N(R)-(CH2)ₖ-, -S(O)₂N(R)-(CH2)ₖ-, -(CH2)ₖ-, -CH₂O-(CH₂)ₖ-, -C(O)S-(CH₂)ₖ-, - CH₂OC(O)N(R)-(CH₂)ₖ-, - CH₂OCH₂CH(OC (O)NH(CH₂)₃-)CH₂OC(O)NH(CH₂)₃-, - C(O)NHCH₂CH[OC(O)NH-]CH₂OC(O)NH-,
   wherein R is hydrogen or CI -4 alkyl, and k is 2 to about 25, preferably k is 2 to about 15, more preferably k is 2 to about 10.
71. A method as referred to in any one of items 53 to 70, wherein the polymer is a thermoplastic.
72. A method as referred to in item 71, wherein the thermoplastic material is selected from the group consisting of polyolefines, polyesters, polyoxymethylene, nylons, and copolymers comprising acrylonitrile, butadiene and styrene.
73 A method as referred to in item 7. wherein the polyolefine is polyethylene or polypropylene.
74. A method as referred to in any one of items 53 to 70 wherein the polymer is a thermoset material.
75. A coated component for a medicinal delivery device comprising a component and a fluorine-containing coating, wherein the fluorine-containing coating comprises two layers, a first polyfluoropolyether-containing layer comprising polyfluoropolyether silane entities of the following Formula Ib:

   R*^{f}*[Q¹-[C(R)₂-Si(O-)₃₋ₓ(R^{1a})ₓ]_{y}]_{z} Ib

   which shares at least one covalent bond with a second non-fluorinated layer comprising entities of the following Formula IIb:

   (-O)₃₋ₘ₋ₙXₙ(R¹)ₘSi-Q-Si(R²)ₖ(X)ₗ(O-)₃₋ₖ₋ₗ IIb

   which in turn shares at least one covalent bond with the component; and wherein:
   R*^{f}* is a monovalent or multivalent polyfluoropolyether segment;
   Q¹ is an organic divalent or trivalent linking group;
   each R is independently hydrogen or a C1-4 alkyl group;
   R^{1a} is a C1-8 alkyl or phenyl group;
   k, 1, m and n are independently 0, 1 or 2, but with the priviso that m+n and k+l are at most 2;
   x is 0 or 1 or 2;
   y is 1 or 2; and
   z is 1, 2, 3, or 4;
   R¹ and R² are independently selected univalent groups, X is a hydrolysable or hydroxy group, m and k are independently 0, 1, or 2 and Q is a divalent organic linking group, comprising a substituted C₂ to C₁₂ hydrocarbyl chain and one or more amine groups.
76. A coated component for a medicinal delivery device as referred to in item 75, wherein the polyfluoropolyether moiety R*^{f}* comprises perfluorinated repeating units selected from the group consisting of -(CₙNF₂ₙO)-,-(CF(Z)O)-, -(CF(Z)CₙF₂ₙO)-, - (CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6.
77. A coated component for a medicinal delivery device as referred to in item 76, wherein the polyfluoropolyether moiety R*^{f}* is C₃F₇O(CF(CF₃)CF₂O)ₚCF(CF₃)-, wherein the average value of p is in the range 3 to 50.
78. A coated component for a medicinal delivery device as referred to in any one of items 75 to 77, wherein z=1.
79. A coated component for a medicinal delivery device as referred to in any one of items 75 to 78, wherein y=1.
80. A coated component for a medicinal delivery device as referred to in item 79, wherein the entity of Formula IIb shares a covalent bond with a polymer surface of the component.
81. A coated component for a medicinal delivery device as referred to in any one of items 79 or 80, wherein Q¹ includes one or more organic linking groups selected from - C(O)N(R)-(CH2)ₖ-, -S(O)₂N(R)-(CH2)ₖ-, -(CH2)ₖ-, -CH₂O-(CH₂)ₖ-, -C(O)S-(CH₂)ₖ-, - CH₂OC(O)N(R)-(CH₂)ₖ-,
   wherein R is hydrogen or C1-4 alkyl, and k is 2 to about 25, preferably k is 2 to about 15, more preferably k is 2 to about 10.
82. A coated component for a medicinal delivery device as referred to in any one of items 75 to 78, wherein y=2.
83. A coated component for a medicinal delivery device as referred to in item 82, wherein the entity of Formula IIb shares a covalent bond with a metal surface of the component, in particular a surface of an aluminium alloy, an iron alloy, or a steel alloy.
84. A coated component for a medicinal delivery device as referred to in any one of items 82 or 83, wherein Q¹ includes as organic linking groups:-

   - CH₂OCH₂CH(OC(O)NH(CH₂)₃-)CH₂OC(O)NH(CH₂)₃-

   or

   - C(O)NHCH₂CH[OC(O)NH-]CH₂OC(O)NH-.
85. A coated component for a medicinal delivery device as referred in any one of items 75 to 84 wherein the component is a component of a metered dose inhaler.
86. A coated component as referred to in item 85, wherein the component is selected from the group consisting of an actuator, an aerosol container, a ferrule, a valve body, a valve stem and a compression spring.
87. A medicinal delivery device assembled from at least one coated component as referred to in any one of items 75 to 86.

Throughout this specification, the word "inhaler" means a device for delivery of a medicament in fluid (or powder) and does not imply that the device requires inhalation on the part of the patient during delivery. It is known that a medicament may be delivered successfully to the nasal passages by an inhaler without the need for the patient to inhale.

So that the present specification may be more completely understood, reference is made to the accompanying drawings in which:
Figure 1a represents a schematic cross-sectional view of a pressurized metered dose inhaler known in the art and
Figure 1b represents an enlarged view of a portion of the inhaler.
Figure 2a and 2b show the results of Example 3.
Figure 3 represents a schematic cross-sectional view of a metered dose valve.

### Detailed Description

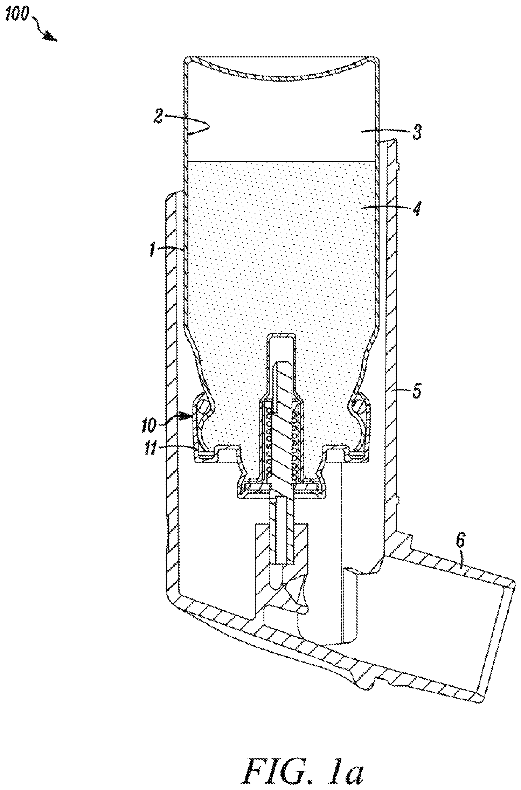

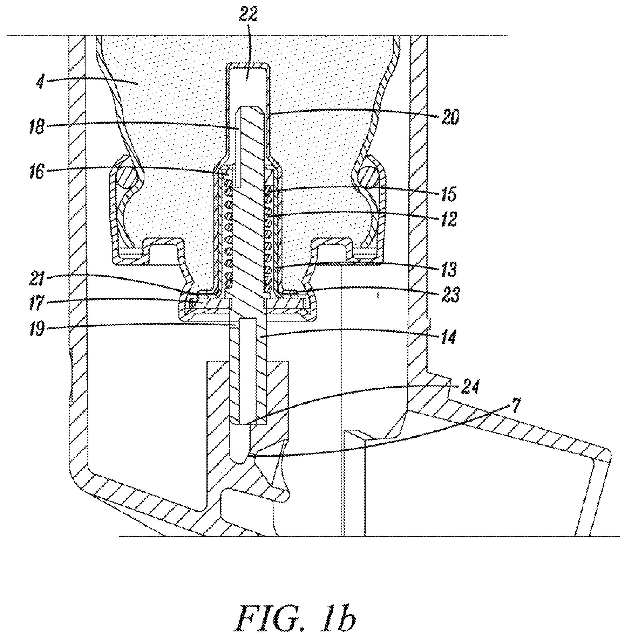

Figure 1a shows a metered dose inhaler 100, including an aerosol container 1 fitted with a metered dose valve 10 (shown in its resting position). The valve is typically affixed, i.e., crimped, onto the container via a cap or ferrule 11 (typically made of aluminium or an aluminium alloy) which is generally provided as part of the valve assembly. Between the container and the ferrule there may be one or more seals. In the embodiments shown in Figures 1a and 1b between the container 1 and the ferrule 11 there are two seals including e.g., an O-ring seal and the gasket seal..

As shown in Figure 1a, the container/valve dispenser is typically provided with an actuator 5 including an appropriate patient port 6, such as a mouthpiece. For administration to the nasal cavities the patient port is generally provided in an appropriate form (e.g., smaller diameter tube, often sloping upwardly) for delivery through the nose. Actuators are generally made of a plastics material, for example polypropylene or polyethylene. As can be seen from Figure 1a, the inner walls 2 of the container and the outer walls 101 of the portion(s) of the metered dose valve located within the container define a formulation chamber 3 in which aerosol formulation 4 is contained.

The valve shown in Figure 1a, and Figure 1b, includes a metering chamber 12, defined in part by an inner valve body 13, through which a valve stem 14 passes. The valve stem 14, which is biased outwardly by a compression spring 15, is in sliding sealing engagement with an inner tank seal 16 and an outer diaphragm seal 17. The valve also includes a second valve body 20 in the form of a bottle emptier. The inner valve body (also referred to as the "primary" valve body) defines in part the metering chamber. The second valve body (also referred to as the "secondary" valve body) defines in part a pre-metering region or chamber besides serving as a bottle emptier.

Referring to Figure 1b, aerosol formulation 4 can pass from the formulation chamber into a pre-metering chamber 22 provided between the secondary valve body 20 and the primary valve body 13 through an annular space 21 between the flange 23 of the secondary valve body 20 and the primary valve body 13. To actuate (fire) the valve, the valve stem 14 is pushed inwardly relative to the container from its resting position shown in Figures 1a and b, allowing formulation to pass from the metering chamber through a side hole 19 in the valve stem and through a stem outlet 24 to an actuator nozzle 7 then out to the patient. When the valve stem 14 is released, formulation enters into the valve, in particular into the pre-metering chamber 22, through the annular space 21 and thence from the pre-metering chamber through a groove 18 in the valve stem past the tank seal 16 into the metering chamber 12.

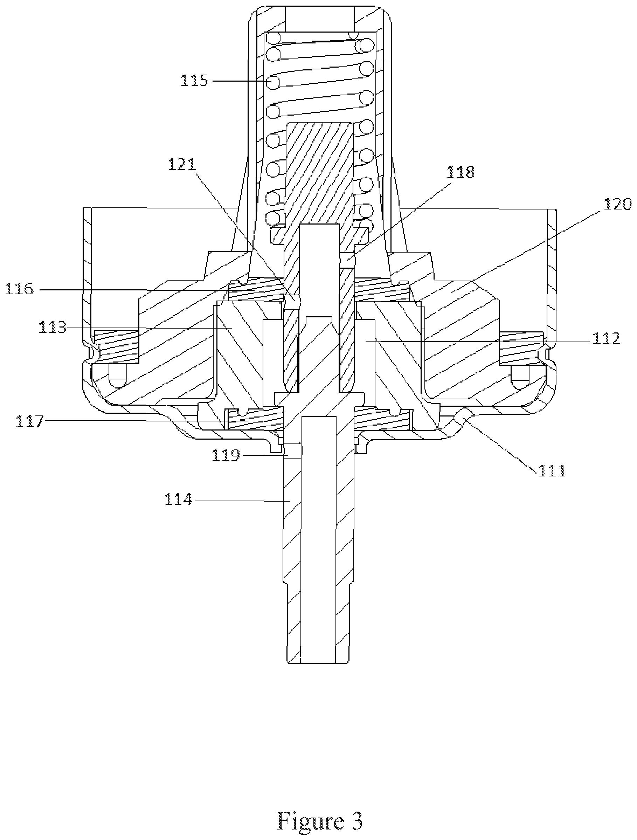

Figure 3 shows a metered dose aerosol valve different to the one shown in Figures 1a, 1b in its rest position. The valve has a metering chamber 112 defined in part by a metering tank 113 through which a stem 114 is biased outwardly by spring 115. The stem 114 is made in two parts that are push fit together before being assembled into the valve. The stem 114 has an inner seal 116 and an outer seal 117 disposed about it and forming sealing contact with the metering tank 113. A valve body 120 crimped into a ferrule 111 retains the aforementioned components in the valve. In use, formulation enters the metering chamber via orifices 121, 118. It's outward path from the metering chamber 112 when a dose is dispensed is via orifice 119.

Depending on the particular metered dose valve and/or filling system, aerosol formulation may be filled into the container either by cold-filling (in which chilled formulation (chilled to temperatures of about -50 to -55°C for propellant HFA 134a-based formulations) is filled into the container and subsequently the metered dose valve is crimped onto the container) or by pressure filling (in which the metered dose valve is crimped onto the container and then formulation is pressure filled through the valve into the container).

The present invention is further illustrated by the following Examples. In this specification, ENFBE refers to solvent ethyl nonafluorobutyl ether, MNFBE refers to methyl nonafluorobutyl ether. The polyfluoropolyethersilane that is referred to as fluorosilane A is a fluorosilane of formula:

(MeO)₃Si(CH₂)₃NHC(O)OCH₂CH[O(O)CHN(CH₂)₃Si(OMe)₃]CH₂NHCOR*^{f2}*

where R^{*f*2} is a moiety of formula CF(CF₃)(OCF₂CF(CF₃))ₖO(CF₂)₂CF₃, k is approximately 5 to 6, but may be in the range 3 to 50, usually 3 to 20, more usually 4 to 10. Fluorosilane B is a fluorosilane of formula:

(MeO)₃Si(CH₂)₃NHC(O)R^{*f*2}

Fluorosilane C is a fluorosilane of formula:
(MeO)₃Si(CH₂)₃OCH₂ R^{*f*2}, in which k is approximately 34.
BTMSPA refers to bis(trimethoxysilylpropyl)amine.
APTMS (ex Sigma) Amino-n-propyltrimethoxysilane
APTES (ex Sigma) Amino-n-propyltriethoxysilane
Primer A is bis(3-trimethoxysilylpropyl) ethylene diamine,
Primer B is bis(triethoxysilyl)ethane,
Primer C is bis(3-triethoxysilylpropyl)amine,
Primer D is bis(3-trimethoxysilylpropyl) N-methyl amine,
Primer E is bis(methyldiethoxysilylpropyl)amine

### Examples

### Deposition test on cans

In the Examples the salbutamol sulphate deposition screening test employed a salbutamol dispersion micronised non-amorphous salbutamol sulphate ultrasonically dispersed in decafluoropentane (1g in 400g). The test was run by employing an Eppendorf pipette to place a 0.3 ml aliquot of the salbutamol dispersion into each can. The cans were then immediately placed on a horizontal rolling mixer (Stuart Scientific model SRT2 operating to rotate cans at 35 rpm) for 3 minutes to allow the dispersion to dry to the surface. The cans were then placed in an air drying oven for 5 minutes at 65°C to fully dry. The cans were then rinsed with 2 aliquots of decafluoropentane (5ml) using a 5 inversion shaking regime with one shake cycle per second.

Controls were uncoated cans to which salbutamol sulphate dispersion had been added and dried but which had not undergone a subsequent rinse step and hence these cans had a deposit level that represented the maximum level possible in the test. Other cans had undergone a subsequent rinse step and hence cans which showed a low level of salbutamol deposition in this test were showing that the deposited salbutamol could be easily washed away and were therefore deemed to have good release/non-stick performance.

The residual deposited salbutamol was quantitatively transferred and assayed by u.v. spectrophotometry. For each sample, the amount assayed was divided by the corresponding amount for the controls and expressed as a percentage. Unless otherwise stated, 3 samples were taken for each determination.

### Example 1

### Method

### Washing of Cans

Cans were immersed in HFE72DE at its boiling temperature (43°C) for 7 minutes. They were allowed to drain for 2 minutes, and then re-immersed for 3 minutes with ultrasonic agitation. These were allowed to drain for a further 4 minutes then dried at ambient conditions for 7 minutes.

### Preparation of primed and unprimed cans

Washed aluminium cans were fill and drain coated with primer solution (BTMSPA 0.2g in isopropanol (109.5g)) by transfer filling brimful into a first can in a first row of 10 cans, allowing 60 s solution contact time, then transferring and topping up from primer solution similarly to the subsequent cans in the row. Meanwhile primer solution was transfer filled into a first can of a second row of 10 cans, and the process repeated as for the first row of cans. Altogether 6 rows of cans were treated, giving a total of 60 cans. The cans were placed inverted to drain on a laboratory wipe, each was shaken and blown dry with compressed air and then placed in an oven for 30 minutes at 60°C.

A similar quantity of washed cans was not primed.

### Coating of cans with fluorosilane A solution for spray coating

A reservoir bottle of 1% w/w fluorosilane A (13.5g) in ENFBE (1355g) was prepared.

An automated spray coater was employed to spray a sequence of cans. The primed and unprimed cans were presented to the sprayer alternately to eliminate any possible systematic trend in the coating efficiency.

In the automated spray coater, spraying liquid was pumped from a reservoir bottle through a spray nozzle mounted to spray vertically upwards for a programmed duration at a spraying station. Each can to be sprayed was delivered inverted to the spraying station, then allowed to drain. By suitable adjustment of the spraying pressure and duration, the optimum coating was achieved when the cans were delivered with a maximum load that did not drip excess coating solution upon draining. 8 cans for each experimental condition were coated. Results are reported to include samples from either side of the optimum. Furthermore, runs were included with 2 passes of the sprayer, allowing 60 s draining between passes, and a run with 3 short duration sprays adding up to the optimum duration for a single spray.

After completion of all coating runs, with draining complete, the cans were placed inverted into a single stainless steel coating tray and placed into the centre of the coating oven at 120°C for 30 minutes. The cans were then removed, left to cool down and then placed into a large polythene bag for storage.

### Testing

Each can was subjected to the deposition test for cans.

### Results

Certain of the deposition cans were tested using the deposition test for cans. Results are reported in Table 1.

**Table 1**

| Primed | No. of passes | No. of sprays | Spray duration (ms) | observation of draining | Salbutamol Sulphate deposition test result (% of control) |
|---|---|---|---|---|---|
| Yes | 1 | 1 | 350 | Single drip from every can | 3 |
| Yes | 1 | 1 | 250 | No dripping | 4 |
| Yes | 2 | 1 | 350 | Occasional dripping | 3 |
| Yes | 2 | 1 | 250 | No dripping | 3 |
| Yes | 1 | 1 | 300 | Only one drip seen in total | 4 |
| Yes | 1 | 3 | 3 times 100 | No dripping | 3 |
| No | 1 | 1 | 350 | Single drip from every can | 5 |
| No | 1 | 1 | 250 | No dripping | 5 |

With reference to Table 1, the data show that all fluorosilane The spray coated cans both with and without the primer show very low deposition values. The primed cans show marginally better deposition performance than the unprimed cans in this study. The uniformly low deposition data across the study indicate that the spray coating process is robust within the coating variable ranges tested.

### Example 2 and Comparative Example 2A

Example 2 involved coating and performance testing of stainless steel valve components.

### Components used

Metering tanks, springs, bottle emptiers, inner and outer seals, valve stems and ferrules were assembled for making valves as shown in Fig. 1a, 1b with a metering volume of 63µl.

### Washing the Components

1000 metering tanks were placed in a stainless steel vessel with lid and ENFBE (1 litre) was added. The components were agitated and then left with the lid on for 30 minutes. After 30 minutes the components were strained using a stainless steel strainer and placed back in the vessel to which a further 1 litre of fresh ENFBE was added. Once more the components were agitated and then left to stand for 30 minutes. The components were then strained and then left in the vessel for the final residues of the ENFBE to evaporate. The components were then placed in a polythene bag for storage.

The same procedure as above was applied to 1000 springs with 500ml ENFBE used in place of the 1 litre quantity. The washed springs were then placed in a polythene bag for storage.

### Coating of the washed components

100 washed springs and 100 washed tanks were placed into a glass 250ml beaker.

### Priming with BTMSPA

BTMSPA (0.3g) was weighed into a 500 ml beaker and isopropanol (164g) was added and the solution mixed. The latter solution was then added to the beaker of components and the components mixed with a spatula then left to stand for 30 seconds. The fluid and components were then poured back into the 500 ml beaker via a stainless steel sieve thereby collecting the coated components in the sieve. The components were then poured out on to a laboratory wipe and were dried off using a hot air blower. The components were returned to the glass beaker and were then placed in an air drying oven at 60 °C for 15 minutes.

### Fluorosilane coating

The primed components were left to cool down and equilibrate with ambient air for 1 hour.

ENFBE (300g) was added to fluorosilane A (3.0g) in a 500ml beaker and the solution was mixed. The solution was the poured into the beaker of primed components and the components were stirred with a spatula. The components were then left to sit for 30 seconds in the solution after which time they were drained via a stainless steel sieve back into the 500 ml beaker hence collecting the components in the sieve. The components were then transferred to a glass beaker and placed into an oven at 120°C for 30 minutes to cure the fluorosilane. The coated components were assembled into inhaler valves as shown in Figs. 1a,1b.

Similarly, the uncoated components (Comparative Example 2A) were also assembled into inhaler valves as a control.

### Product filling

The coated test valves and control valves were incorporated into metered dose inhaler and used to deliver a formulation of micronised salbutamol sulphate suspended in HFA134a. The target dose of the product was 100µg per actuation and the cold filling technique was employed for the filling process.

### Product testing

Both the coated (Example 2) and the uncoated (Comparative Example 2A) valve product lots were actuated through life to shot number 120, after which point the test inhalers were chilled down, the valves removed and the valve components disassembled and assayed for salbutamol sulphate residue. The assay was done by uv spectrophotometry of quantitative washings from each component made up to a suitable volume with washing solvent. The assay results are shown in table 2, below.

**Table 2**

| Component | Mass of salbutamol sulphate deposited on component at end of life (mg) | |
|---|---|---|
| | Coated Example 2 | Uncoated Comparative Example 2A |
| Stem and seal | 0.25 | 0.47 |
| Spring | 0.09 | 0.38 |
| Tank | 0.11 | 0.25 |
| Bottle emptier | 0.03 | 0.11 |

The data in Table 2 show that the valves incorporating stainless steel springs and stainless steel tanks coated with BTMSPA followed by fluorosilane A in Example 2 show a very large reduction in component salbutamol sulphate deposition levels compared to the uncoated controls in Comparative Example 2A. Although only the springs and the tanks were coated, coating appears to have had a further beneficial effect in terms of lowered deposition also on the stem and seal and the bottle emptier components of the coated valves. This effect is likely due to reduced deposition at the interface points between uncoated and coated components compared to that seen at the interface points between components that are all uncoated.

### Example 3

### Preparation

### Coating of the washed components

400 washed springs were placed into a 500ml beaker and 400 washed 63 µl metering tanks were placed into a second 500ml beaker. The washing procedure was as for example 2.

### Priming

BTMSPA (0.4g) was weighed into a 500 ml beaker and isopropanol (219g) was added and the solution mixed. The latter solution was then added to the beaker of springs and mixed well with a stainless steel spatula and left to stand for 30 seconds. The fluid and components were then poured out into the beaker containing the metering tanks and the latter were then mixed well and left to stand for 30 seconds. The metering tanks and fluid contents were then poured through a sieve into a third beaker. The still wet metering tanks and springs were separated out onto two separate laboratory wipes and left in the fume cupboard with fast air flow for 10 minutes until all the isopropanol had evaporated. The components were then transferred into dry 500ml glass beakers and placed into the air drying oven for 15 minutes at a temperature of 60°C.

### Fluorosilane coating

The primed metering tanks and springs were removed from the air drying oven and poured out onto laboratory wipe in the fume cupboard to allow to cool and to equilibrate with atmospheric moisture for 30 minutes.

ENFBE (400g) was added to fluorosilane A (4.0g) in a 500ml beaker and the solution was mixed. The latter solution was then poured into the beaker of primed springs components and the components were stirred with a spatula. The components were then left to sit for 30 seconds in the solution after which time they were drained via a stainless steel sieve back into the 500 ml beaker containing the metering tanks. After 30 seconds the metering tanks were sieved to isolate them and each beaker of components was air dried on a laboratory wipe with the aid of a hot air blower, then placed into the oven at 120°C for 30 minutes to cure the fluorosilane.

Valves as shown in Figs 1a,1b were assembled from the above metering tanks and springs, by incorporating seals, a bottle emptier and a ferrule for each set and crimping. Valves as shown in Figs 1a,1b were prepared for comparison as above, with the exception that the priming and fluorosilane coating steps were omitted.

A suspension formulation of fluticasone propionate was made up in propellant (1%w/w ethanol + 99%w/w HFA134a). Aluminium cans were filled to deliver 120 shots via 63µl valve, by cold transferring aliquots of the suspension, and valves prepared as above were crimped on to provide aerosol units.

### Testing

Each aerosol unit to be tested was placed into a plastic actuator (the discharge actuator). The aerosol unit was shaken with a gentle rocking action through 180° inversion for at least 10 s and a shot was fired to waste. The valve was immediately released, and these steps repeated, once using the discharge actuator and again twice using a fresh plastic actuator (the test actuator).

An USCA (Unit Spray Sample Collection) apparatus as described in US Pharmacopoeia vol. 29 (2006) section <601> was set up. An aerosol unit which had been through firing shots to waste as described above was immediately attached and a pair of shots fired into an USCA Medication Delivery collection tube with a filter (USCA tube), according to the procedure for the apparatus. Each pair of shots from the unit was fired into a separate USCA tube. The drug was quantitatively transferred from each USCA tube for analysis by hplc.

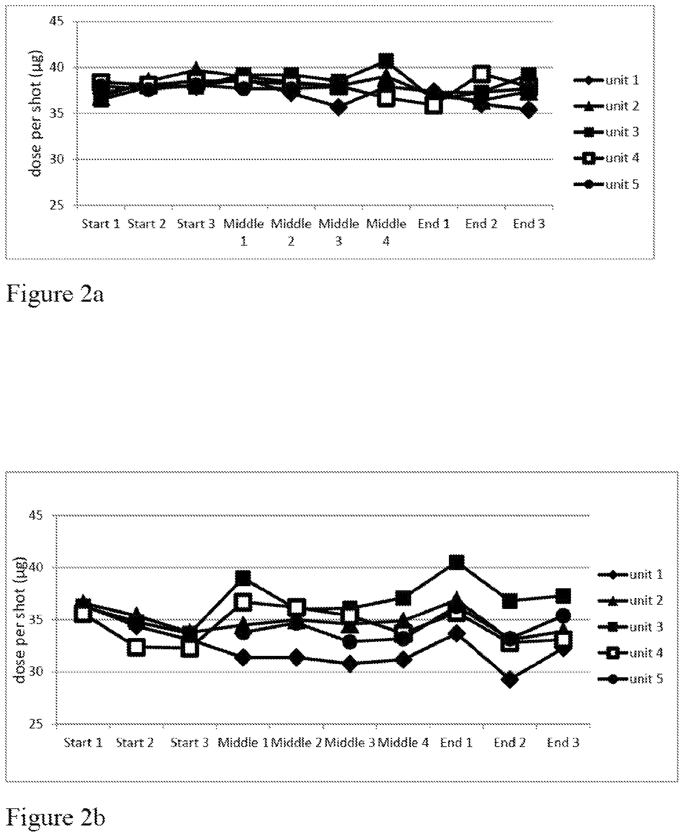

The test regimen, following on from the firing to waste described above and carried out on 5 units for each of the 2 valve types, was as follows:
3 pairs of shots representing the start of unit life through the test actuator (referred to by their sample number as start 1, start 2 and start 3 in Figure 2a, 2b)
48 shots fired to waste through the discharge actuator
2 shots fired to waste through the test actuator
4 pairs of shots representing the middle of unit life through the test actuator (referred to as middle 1, middle 2, middle 3 and middle 4 in Figure 2a, 2b)
48 shots fired to waste through the discharge actuator
2 shots fired to waste through the test actuator
3 pairs of shots representing the end of unit life through the test actuator (referred to as end 1, end 2 and end 3 in Figure 2a, 2b)

### Results

**Table 3**

| | Units with coated springs and metering tanks | Units with uncoated springs and metering tanks |
|---|---|---|
| Start shots mean | 38.0µg | 34.9µg |
| Middle shots mean | 38.3µg | 345µg |
| End shots mean | 37.2µg | 34.7µg |
| Start shots relative standard deviation | 2.0% | 6.5% |
| Middle shots relative standard deviation | 2.8% | 6.3% |
| End shots relative standard deviation | 2.9% | 7.7% |
| Overall mean and relative standard deviation | 37.9 µg ± 2.8% | 34.7 µg ± 6.7% |

A graph of µg fluticasone released per shot is shown in Figure 2a as a function of through life position and sample number for Example 3, showing a high level of consistency using the valve assembled with coated components. For comparison Figure 2b shows the corresponding results for the valve with uncoated components. The results indicate that coating according to the invention is highly advantageous because it results in less dose to dose variability of the medicament, and less unit to unit variability.

### Example 4

### Preparation

19 ml aluminium cans were washed with HFE72DE as in Example 1.
4A - BMSTPA (0.05g) was dissolved in isopropanol (27.4g). The solution was transferred sequentially brimful into 6 aluminium cans, allowing a solution contact time of 60 s per can. The cans were then drained and placed in an oven at 60°C for 2 hours, to complete priming. fluorosilane A (0.5g) was weighed into a plastic beaker and made up to 50g with MNFBE. The solution was transferred sequentially brimful into 3 primed aluminium cans, allowing a solution contact time of 30 s per can. These cans were drained and placed in an oven at 120°C for 15 minutes, to effect curing.
4B - BMSTPA (0.05g) and fluorosilane A (0.5g) were dissolved in ethanol (27.4g) in a plastic beaker. The solution was transferred sequentially brimful into 3 cleaned aluminium cans, allowing a solution contact time of 60 s per can. These cans were drained and placed in an oven at 120°C for 15 minutes, to effect curing.
4C - BMSTPA (0.05g) was dissolved in ethanol (27.4g). The solution was transferred sequentially brimful into 3 aluminium cans, allowing a solution contact time of 5 s per can. The cans were then drained and placed in an oven at 120°C for 15 minutes, to complete priming. fluorosilane A (0.5g) was dissolved in ethanol (27.4g). The solution was transferred sequentially brimful into 3 primed aluminium cans, allowing a solution contact time of 60 s per can. These cans were drained and placed in an oven at 120°C for 15 minutes, to effect curing and complete the coating.
4D - fluorosilane A (0.5g) was dissolved in ethanol (27.4g). The solution was transferred sequentially brimful into 3 cleaned aluminium cans, allowing a solution contact time of 60 s per can. These cans were drained and placed in an oven at 120°C for 15 minutes, to effect curing and complete the coating.

### Testing

The deposition test for cans described earlier was carried out.

### Results

**Table 4**

| Sample | Description | % deposition |
|---|---|---|
| 4A | HFE72DE cleaned+BTMSPA primed + fluorosilane A coated | 1 |
| 4B | HFE72DE cleaned+(fluorosilane A coated+BTMSPA crosslinked) | 7 |
| 4C | HFE72DE cleaned+BTMSPA fast primed + fluorosilane A coated | 3 |
| 4D | HFE72DE cleaned+ fluorosilane A coated | 5 |

### Example 5

### Preparation

10 ml Stainless steel cans were washed with ENFBE as follows. 65 cans were periodically agitated in ENFBE (2 liters) with a contact time of 1 hour, then drained and allowed to dry at ambient conditions.

Priming, where used, was carried out by taking 20 washed cans and contacting with a solution of BTMSPA (0.1 g) in isopropanol (54.8 g) for 30 s, allowing to drain, then placing in an oven at 60°C for 1 hour.

Fluorosilane coating, where used, was carried out by taking 6 cans a filling with fluorosilane A (0.5 g) in MNFBE (50 g) for 30 s, allowing to drain, then placing in an oven at 120°C for 15 minutes.

### Testing

6 cans of each test condition were subjected to the Deposition test for cans as follows:
5A - washed only
5B - washed and primed
5C - washed, primed and coated
5D - washed and coated

### Results

**Table 5**

| Sample | % deposition |
|---|---|
| 5A | 84 |
| 5B | 88 |
| 5C | 2 |
| 5D | 5 |

The results indicate that the effect of coating was improved by a previous priming, while priming alone was ineffective.

It is to be understood that the specification is not limited to the embodiments described above and that various modifications can be made without departing from the principles or concepts of the specification.

Actuators and inhalers as claimed in the specification may include any feature described herein separately or in combination with any other feature(s), if necessary with appropriate modification of other features, as would be readily apparent to the skilled person.

### Example 6

Coatings were applied to the internal surfaces of PET vials and their effectiveness assessed by the deposition test referred to earlier.

### Method

BTMSPA, APTMS and APTES were each prepared individually as solutions of 0.1g in ethanol (50g). Each primer solution was then used to prime 3 PET vials using the fill and drain technique with 30 seconds contact time and then draining the vials upside down for 2 minutes then placing upright for 30 minutes to equilibrate then curing at 55°C for 30 minutes in an oven with a wet lab wipe to boost the moisture level. Each of the 3 samples above was then left to equilibrate with room temperature and humidity for 4 hours prior to ECC7000 coating.

Each vial was then fill and drain coated with a solution of Fluorocarbon B (10% solution) (1.0g) in MNFBE (99g). The vials were left to equilibrate with ambient air for 30 minutes and then cured at 55°C for 30 minutes in an oven with a wet lab wipe to boost the moisture level.

### Results

The details of any primer and the top coat are given in Table 6 along with the results of the deposition test.

**Table 6**

| Sample designation | Primer | Top coat | % deposition |
|---|---|---|---|
| 6A | BTMSPA | Fluorosilane B | 0, 0, 0 |
| 6B | APTMS | Fluorosilane B | 21, 28, 25 |
| 6C | APTES | Fluorosilane B | 28, 26, 30 |
| 6D | none | Fluorosilane B | 55, 64, 55 |
| Uncoated PET vials | none | none | 80, 82, 81 |

### Example 7

Further examples of coatings applied to the internal surfaces of PET vials were assessed by the deposition test.

### Method

A top coating solution of Fluorosilane B (10%w/w) was prepared from Fluorosilane B (2g) in MNFBE (18g).

A top coating solution of Fluorosilane C (8%w/w) was prepared from a Fluorosilane C 20%w/w solution (13.5g) in MNFBE (20.2g).

### Priming the PET vials

PET vials (27) were fill and drain coated in a solution of BTMSPA (0.2g) in dehydrated ethanol (99.8g). The vials were then placed open end down onto lab wipe for 5 minutes, to drain excess coating solution, before being placed upright to equilibrate with ambient air (22°C 34% RH) for 30 minutes. The vials were then cured in an oven at 60°C for 30 minutes.

### Top coating of PET vials

The 27 BTMSPA primed PET vials were separated into 9 sets of 3 vials and were coated as shown in the table below employing the fill and drain approach with a 30 seconds contact time. All vials were placed open end down for 5 minutes to drain the coating solution, before being stored open end up for 30 minutes to equilibrate at ambient temperature and humidity (22°C 34% RH). The vials were then oven cured at 55°C for 30 minutes.

A comparative experiment was performed at the same time in which a primer solution and top coat solution were mixed together and applied as a single coating. Another comparative experiment compared top coating without primer coating.

**Table 7a**

| Sample designation | Description of top coating solution | % deposition |
|---|---|---|
| 7A | Fluorosilane A 10% solution (0.5g) in MNFBE (99.5g) | 19, 19, 22 |
| 7B | Fluorosilane A 10% solution (1.0g) in MNFBE (99.0g) | 21, 18, 23 |
| 7C | Fluorosilane A 10% solution (2.0g) in MNFBE (98.0g) | 12, 15, 19 |
| 7D | Fluorosilane B 10% solution (0.5g) in MNFBE (99.5g) | 0, 0, 0 |
| 7E | Fluorosilane B 10% solution (1.0g) in MNFBE (99.0g) | 0, 0, 0 |
| 7F | Fluorosilane B 10% solution (2.0g) in MNFBE (98.0g) | 0, 0, 1 |
| 7G | Fluorosilane C 8% solution (0.62g) in MNFBE (99.4g) | 1, 2, 2 |
| 7H | Fluorosilane C 8% solution (1.25g) in MNFBE (98.75g) | 0, 0, 1 |
| 7I | Fluorosilane C 8% solution (2.5g) in MNFBE (97.5g) | 0, 0, 1 |
| Uncoated PET vials | none | 80, 82, 81 |

**Table 7b**

| Sample designation | Description of coating | % deposition |
|---|---|---|
| 7J | As for 7F, but as single coat of mixed primer and top coat solutions | 53, 57, 58 |
| 7K | As for 7E, but with no priming coat | 59, 62, 58 |

### Results

The details of top coat for examples are given in Table 7a along with results of the deposition test, and a comparison where no coating was applied. Table 7b gives some further comparative tests with alternative coatings and deposition test results.

The vials with BTMSPA prime and Fluorosilane A top coat showed acceptably low deposition. Those with BTMSPA prime and Fluorosilane B or C top coat showed virtually no deposition at all.

### Example 8

Further examples of coatings applied to the internal surfaces of PET vials were assessed by the deposition test.

### Method

Each primer (A to E and also BTMSPA) (0.1g) was separately dissolved in isopropanol (50g).

PET vials (n=3, for each sample designation) were then fill and drain coated with each primer solution using a 30 seconds contact time. The vials were then oven cured at 60°C for 30 minutes. The primed vials were then left overnight.

Fluorosilane B (1.0g) was dissolved in MNFBE (9g) to make a 10%w/w solution. 10%w/w solution (1.0g) was then dissolved in MNFBE (99g). All vials were fill and drain coated for 30 seconds and then oven cured for 30 minutes at 0°C.

Details of the coatings applied and results of the salbutamol sulphate deposition test are tabulated in Table 8.

**Table 8**

| Sample designation | Primer | Top coat | % deposition |
|---|---|---|---|
| 8A | Primer A | Fluorosilane B | 1.4, 0.4, 0.7 |
| 8B | Primer B | Fluorosilane B | 56.6, 54.8, 61.8 |
| 8C | Primer C | Fluorosilane B | 0.3, 0.3, 0.4 |
| 8D | Primer D | Fluorosilane B | 0.7, 0.3, 0.3 |
| 8E | Primer E | Fluorosilane B | 2.2, 4.2, 2.4 |
| 8F | BTMSPA | Fluorosilane B | 0.1, 0.3, 0.3 |
| 8G | none | Fluorosilane B | 58.4, 50.7, 57.9 |

### Results

Very low depositions similar to those seen in the previous example were repeated here when other primers were used, except where the primer had an unsubstituted alkyl linking group (Primer B).

### Example 9

A modified coating process for applying coatings to the internal surfaces of PET vials was assessed by the deposition test.

### Methods

Priming solution was prepared from BTMSPA (0.2g) dissolved in ethanol and made up to 99.8g.

Fluorosilane B 10%w/w solution (1.0g) was added to MNFBE (99g) to prepare a top coating solution.
The following samples were prepared:
9A - PET vials (3) were fill and drain coated in priming solution. The vials were then placed open end down onto lab wipe for 5 minutes, to drain excess coating solution, before being placed upright to equilibrate with ambient air (22°C 34% RH) for 30 minutes. The vials were then cured in an oven at 60°C for 30 minutes. Top coating solution was applied by filling and draining with a 30 seconds contact time. All vials were placed open end down for 5 minutes to drain the coating solution, before being stored open end up for 30 minutes to equilibrate at ambient temperature and humidity (22°C 34% RH). The vials were then oven cured at 60°C for 30 minutes.
9B - PET vials (3) were fill and drain coated in priming solution. The vials were then quickly air-dried. Top coating solution was applied by filling and draining with a 30 seconds contact time. All vials were placed open end down for 5 minutes to drain the coating solution, before being stored open end up for 30 minutes to equilibrate at ambient temperature and humidity (22°C 34% RH). The vials were then oven cured at 60°C for 30 minutes.
9C - No primer was used. Top coating solution was applied by filling and draining with a 30 seconds contact time. All vials were placed open end down for 5 minutes to drain the coating solution, before being stored open end up for 30 minutes to equilibrate at ambient temperature and humidity (22°C 34% RH). The vials were then oven cured at 60°C for 30 minutes.

### Results

**Table 9**

| Sample designation | Primer | Top coat | | % deposition |
|---|---|---|---|---|
| 9A | BTMSPA | Fluorosilane B | Prime coat heat-cured | 0, 1, 0, |
| 9B | BTMSPA | Fluorosilane B | Prime coat air-dried | 2, 1, 1, |
| 9C | none | Fluorosilane B | No prime coat | 51, 46, 53 |

Details of the primer and top coat used and any method variations are provided in table 9, along with the results of the deposition test. The prime and top coating may be cured in a single curing step.

### Example 10

Metering valves as shown in Figs. 1a and 1b were assembled with various coatings, and inhalers were prepared in which the metering valves were crimped onto cans containing a suspension formulation in an HFA propellant system. After dispensing the contents, the deposition levels of a medicament on components of the valves were assayed.

### Methods

### Priming of metal springs and tanks

A priming solution of BTMSPA (0.2g) dissolved in HFE72DE (168g) was prepared. 120 springs and 120 tanks, which had previously been washed as in Example 2, were dip coated in the coating solution and then oven cured at 120°C for 30 minutes.

### Priming of plastic stems

80 PBT plastic stems were washed in ENFBE as in Example 2.

A priming solution of BTMSPA (0.1g) dissolved in dehydrated ethanol (50g) was prepared. 80 washed PBT plastic stems were dip coated in the coating solution and then oven cured at 55°C for 30 minutes.

### Top coating of primed springs and tanks with Fluorosilane A

A top coating solution of Fluorosilane A (2.0g) dissolved in HFE72DE (168g) was prepared. 80 primed tanks and 80 primed springs were dip coated for 30 seconds in the solution. The components were then oven cured at 120°C for 30 minutes.

### Top coating of primed springs and tanks with Fluorosilane B

A top coating solution of Fluorosilane B (10%) (1.0g) dissolved in MNFBE (84g) was prepared. 40 primed tanks and 40 primed springs were dip coated for 30 seconds in the solution. The components were then oven cured at 120°C for 30 minutes.

### Top coating of primed plastic stems

A top coating solution of Fluorosilane A (1.0g) dissolved in MNFBE (99g) was prepared.

A top coating solution of Fluorosilane B (10%) (1.0g) dissolved in MNFBE (99g) was prepared.

40 of the BTMSPA-primed plastic stems were dip coated in each solution above as specified in Table 10a. The coated stems were then dried on lab wipe and then oven cured at 55°C for 30 minutes.

### Preparation of test samples

Valves were constructed as in Table 10a below. For each valve lot 10A-10F, five valves were constructed and tested in inhaler canisters (n=5). The springs and tanks of the test samples and the controls were coated, while the bottle emptiers were left uncoated. Use of talc or Magnesium Stearate on the seals (inner tank seal and diaphragm seal) and/or silicone oil lubrication of the stem and seals sub-assembly during valve assembly was done to ensure that the valves functioned reliably when sealed onto inhaler canisters. Nevertheless, controls with and without the seal lubrication and silicone oil lubrication were performed. The assembled valves were then placed on inhaler canisters containing medicament suspended in an HFA propellant formulation.

The inhalers were actuated repeatedly to exhaust the contents through the valves, then the valves were removed and carefully dismantled. The medicament deposits on the separate components were assayed by washing them off in a suitable solvent and sampling to an hplc.

**Table 10a**

| Valve lot | Description | Springs Top coating | Tanks Top coating | Plastic stems Top coating |
|---|---|---|---|---|
| 10A | Uncoated stem, control with talc and silicone oil | Fluorosilane A | Fluorosilane A | Uncoated |
| 10B | Uncoated stem, control valve without talc and silicone oil | Fluorosilane A | Fluorosilane A | Uncoated |
| 10C | Coated stem, test A stem with talc and silicone | Fluorosilane A | Fluorosilane A | Fluorosilane A |
| 10D | Coated stem, test A stem with Magnesium Stearate dusting of seals | Fluorosilane A | Fluorosilane A | Fluorosilane A |
| 10E | Coated stem, test B stem with talc | Fluorosilane B | Fluorosilane B | Fluorosilane B |
| 10F | Coated stem, test B stem with Magnesium Stearate dusting of seals | Fluorosilane B | Fluorosilane B | Fluorosilane B |

Magnesium Stearate dusting was carried out as disclosed in WO2012/173971 (Experiment 2 dry coating): about 2½ mg Magnesium stearate per gram of seals was used for the inner tank seals; about 1 mg Magnesium stearate per gram of seals was used for the diaphragm seals.

### Results

The levels of deposited medicament on each of the components following exhaustion of the contents of the inhalers is shown in Table 10b. The average cumulative deposition for 3 components (spring, tank and plastic stem) is presented alongside the individual values for the components.

**Table 10b**

| Valve lot | Medicament deposition (mg) per spring | Medicament deposition (mg) per tank | Medicament deposition (mg) per plastic stem | Medicament deposition (mg) 3 component average |
|---|---|---|---|---|
| 10A | 157, 122, 121, 186, 148 | 93, 123, 102, 122, 100 | 139, 185, 145, 155, 156 | 410.8 |
| 10B | 149, 180, 123, 145, 111 | 81, 85, 88, 73, 97 | 152, 144, 135, 144, 147 | 370.8 |
| 10C | 49, 46, 41, 41, 42 | 64, 106, 104, 85, 79 | 80, 83, 58, 67, 51 | 199.2 |
| 10D | 50, 45, 63, 43, 55 | 59, 92, 86, 82, 89 | 65, 95, 60, 61, 50 | 199.0 |
| 10E | 24, 22, 56, 18, 25 | 37, 64, 76, 41, 48 | 77, 61, 73, 75, 59 | 151.2 |
| 10F | 24, 54, 35, 48, 46 | 61, 55, 54, 50, 58 | 63, 64, 43, 90, 85 | 166.0 |

The 3 component average medicament deposition was improved by coating the plastic stem relative to both controls (10A and 10B), as was the medicament deposition on the plastic stem itself.

Even though the stems and springs were coated using primer and fluorosilane in both test and control runs, medicament deposition on the spring and tank was still generally improved by coating the plastic stem.

### Example 11

Valves as shown in Figure 3 were assembled using components that had been coated. The coated components were made from polybutylene terephthalate (PBT). The valves were crimped onto cans containing a suspension formulation, and tested for drug deposition after dispensing the doses.

### Components used

Metering tanks, springs, valve bodies, inner and outer seals, valve stems and ferrules were gathered for making valves as shown in Fig. 3 with a metering volume of 50µl.

### Priming of metering tanks, stems, springs and valve bodies with BTMSPA

20 metering tanks, stems, springs, valve bodies were immersed with agitation for 30 seconds in a primer solution comprising BTMSPA (0.2g) in dehydrated ethanol (100g). The components were strained out then air dried and then placed in an air drying oven for 30 minutes at 60°C to cure the priming coating.

### Fluorosilane coating

The primed components were left to cool down and equilibrate with ambient air overnight.

10 sets of metering tanks, stems, springs, valve bodies were each coated with one of each of 2 fluorosilanes as follows:
A top coating solution of Fluorosilane B (10%) (1.0g) dissolved in MNFBE (99g) was prepared for use in Example 11B.

A top coating solution of Fluorosilane C (0.2%) in MNFBE was prepared for use in Example 11C.

The above components sets were placed into individual 250ml glass reagent bottles and contacted for 3 minutes with occasional agitation with the appropriate fluorosilane solution. The components were then strained out air dried and then cured at 60°C for 16 hours.

The coated components were assembled into inhaler valves as shown in Fig. 3.

Similarly, the uncoated components were also assembled into inhaler valves as controls.

### Product filling

The coated test valves and control valves were incorporated into metered dose inhaler and used to deliver a formulation of micronised salbutamol sulphate suspended in HFA134a. The target dose of the product was 100µg per actuation and the cold filling technique was employed for the filling process.

### Product testing

Both the coated (Examples 11B and 11C) and the uncoated (Comparative Example 11A) valve product lots were actuated through life to shot number 120, after which point the test inhalers were chilled down, the valves removed and the valve components disassembled and assayed for salbutamol sulphate residue (n=5 for each valve lot).

The assay was done by uv spectrophotometry of quantitative washings from each component made up to a suitable volume with washing solvent. The assay results are shown in table 11.

**Table 11**

| Valve lot | Treatment of components | Residual drug deposition (mg) | | |
|---|---|---|---|---|
| | | Tank | Stem | body |
| 11A | Uncoated and unwashed components | 0.218 0.161 | 0.182 0.199 | 0.129 0.146 |
| | | 0.197 | 0.192 | 0.144 |
| | | 0.211 | 0.146 | 0.122 |
| | | 0.262 | 0.153 | 0.213 |
| | Average ± Std deviation | 0.210±0.037 | 0.174±0.024 | 0.151±0.036 |
| 11B | Coated with BTMSPA + Fluorosilane B | 0.163 0.137 | 0.149 0.166 | 0.139 0.060 |
| | | 0.142 | 0.136 | 0.077 |
| | | 0.233 | 0.173 | 0.082 |
| | | 0.144 | 0.146 | 0.125 |
| | Average ± Std deviation | 0.164±0.040 | 0.154±0.015 | 0.097±0.034 |
| 11C | Coated with BTMSPA + Fluorosilane C | 0.070 0.099 | 0.111 0.151 | 0.069 0.067 |
| | | 0.110 | 0.163 | 0.067 |
| | | 0.137 | 0.110 | 0.075 |
| | | 0.136 | 0.137 | 0.098 |
| | Average ± Std deviation | 0.110±0.028 | 0.134±0.024 | 0.075±0.013 |

## Claims

1. A method of making a component for a medicinal delivery device, the method comprising
a) providing a component of a medicinal delivery device,
b) providing a primer composition comprising a silane having two or more reactive silane groups separated by an organic linker group,
c) providing a coating composition comprising an at least partially fluorinated compound,
d) applying the primer composition to at least a portion of the surface of the component,
e) applying the coating composition to the portion of the surface of the component after application of the primer composition.

2. A method as claimed in claim 1, wherein the silane having two or more reactive silane groups is of formula
X₃₋ₘ(R¹)ₘSi-Q-Si(R²)ₖX₃₋ₖ
wherein R¹ and R² are independently selected univalent groups, X is a hydrolysable or hydroxy group, m and k are independently 0, 1, or 2 and Q is a divalent organic linking group.

3. A method as claimed in claim 2, wherein Q is of formula - (CH₂)ᵢ-A-(CH₂)ⱼ - wherein A is NRⁿ, O, or S; i and j are independently 0, 1, 2, 3 or 4 and wherein Rⁿ is H or C₁ to C₄ alkyl.

4. A method as claimed in any preceding claim, wherein the partially fluorinated compound is a polyfluoropolyether silane of formula
R^{f}Q¹ᵥ[Q²_{w}-[C(R⁴)₂-Si(X)₃₋ₓ(R⁵)ₓ]_{y}]_{z}
wherein:
R*^{f}* is a polyfluoropolyether moiety;
Q¹ is a trivalent linking group;
each Q² is an independently selected organic divalent or trivalent linking group;
each R⁴ is independently hydrogen or a C₁₋₄ alkyl group;
each X is independently a hydrolysable or hydroxyl group;
R⁵ is a C1-8 alkyl or phenyl group;
v and w are independently 0 or 1, x is 0 or 1 or 2; y is 1 or 2; and z is 2, 3, or 4.

5. A method as claimed in claim 4, wherein the polyfluoropolyether moiety R*^{f}* comprises perfluorinated repeating units selected from the group consisting of -(CₙF₂ₙO)-,-(CF(Z)O)-, (CF(Z)CₙF₂ₙO)-, -(CₙF₂ₙCF(Z)O)-, -(CF₂CF(Z)O)-, and combinations thereof; wherein n is an integer from 1 to 6 and Z is a perfluoroalkyl group, an oxygen-containing perfluoroalkyl group, a perfluoroalkoxy group, or an oxygen-substituted perfluoroalkoxy group, each of which can be linear, branched, or cyclic, and have 1 to 5 carbon atoms and up to 4 oxygen atoms when oxygen-containing or oxygen-substituted and wherein for repeating units including Z the number of carbon atoms in sequence is at most 6.

6. A method as claimed in any one of the preceding claims, wherein said surface is a metal surface, in particular a surface of an aluminium alloy, an iron alloy, or a steel alloy.

7. A method as claimed in any one of the preceding claims, where said medicinal delivery device is a metered dose inhaler or a dry powder inhaler.

8. A method as claimed in any one of the preceding claims, wherein the component is a component of a metered dose inhaler and the component is selected from the group consisting of an actuator, an aerosol container, a ferrule, a valve body, a valve stem and a compression spring.

9. A method as claimed in any one of claims 1 to 5, 7 and 8, wherein said portion of surface is a polymer surface.

10. A method as claimed in claim 9 wherein the component is at least partly made of said polymer.

11. A method as claimed in any one of claims 9 to 10, wherein the at least partially fluorinated compound is polyfluoropolyether silane of the Formula Ia:
R*^{f}*[Q¹-[C(R)₂-Si(Y)₃₋ₓ(R^{1a})ₓ]_{y}]_{z} Ia
wherein:
R*^{f}* is a monovalent or multivalent polyfluoropolyether moiety;
Q¹ is an organic divalent or trivalent linking group;
each R is independently hydrogen or a C1-4 alkyl group;
each Y is independently a hydrolysable group;
R^{1a} is a C1-8 alkyl or phenyl group;
x is 0 or 1 or 2;
y is 1 or 2; and
z is 1, 2, 3, or 4.

12. A method as claimed in claim 11, wherein the polyfluoropolyether moiety R*^{f}* is C₃F₇O(CF(CF₃)CF₂O)ₚCF(CF₃)-, wherein the average value of p is in the range 3 to 50.

13. A coated component for a medicinal delivery device comprising a component and a fluorine-containing coating, wherein the fluorine-containing coating comprises two layers, a first polyfluoropolyether-containing layer comprising polyfluoropolyether silane entities of the following Formula Ib:
R*^{f}*[Q¹-[C(R)₂-Si(O-)₃₋ₓ(R^{1a})ₓ]_{y}]_{z} Ib
which shares at least one covalent bond with a second non-fluorinated layer comprising entities of the following Formula IIb:
(-O)₃₋ₘ₋ₙ(X)ₙ(R¹)ₘSi-Q-Si(R²)ₖ(X)ₗ(O-)₃₋ₖ₋ₗ IIb
which in turn shares at least one covalent bond with the component; and wherein:
R*^{f}* is a monovalent or multivalent polyfluoropolyether segment;
Q¹ is an organic divalent or trivalent linking group;
each R is independently hydrogen or a C1-4 alkyl group;
R^{Ia} is a C1-8 alkyl or phenyl group;
k, l, m and n are independently 0, 1 or 2, but with the priviso that m+n and k+l are at most 2;
x is 0 or 1 or 2;
y is 1 or 2; and
z is 1, 2, 3, or 4;
R¹ and R² are independently selected univalent groups, X is a hydrolysable or hydroxy group, m and k are independently 0, 1, or 2 and Q is a divalent organic linking group, comprising a substituted C₂ to C₁₂ hydrocarbyl chain and one or more amine groups.

## Patentansprüche

1. Ein Verfahren zum Herstellen einer Komponente für eine medizinische Abgabevorrichtung, das Verfahren umfassend
a) Bereitstellen einer Komponente einer medizinischen Abgabevorrichtung,
b) Bereitstellen einer Primerzusammensetzung, umfassend ein Silan mit zwei oder mehr reaktiven Silangruppen, die durch eine organische Verknüpfungsgruppe getrennt sind,
c) Bereitstellen einer Beschichtungszusammensetzung, umfassend eine zumindest teilweise fluorierte Verbindung,
d) Auftragen der Primerzusammensetzung auf mindestens einen Abschnitt der Oberfläche der Verbindung,
e) Auftragen der Beschichtungszusammensetzung auf den Abschnitt der Oberfläche der Komponente nach dem Auftragen der Primerzusammensetzung.

2. Ein Verfahren nach Anspruch 1, wobei das Silan mit zwei oder mehr reaktiven Silangruppen aus der Formel
X₃₋ₘ(R¹)mSi-Q-Si(R²)ₖX₃₋ₖ besteht
worin R¹ und R² unabhängig voneinander ausgewählte einwertige Gruppen sind, X eine hydrolysierbare oder Hydroxygruppe ist, m und k unabhängig voneinander 0, 1 oder 2 sind und Q eine zweiwertige organische Verknüpfungsgruppe ist.

3. Ein Verfahren nach Anspruch 2, worin Q die Formel -(CH₂)ᵢ-A-(CH₂)ⱼ-besitzt, worin A NRⁿ, O oder S ist; i und j sind unabhängig voneinander 0, 1, 2, 3 oder 4 und worin Rⁿ H oder C₁ bis C₄ Alkyl ist.

4. Ein Verfahren nach einem der vorhergehenden Ansprüche, worin die teilweise fluorierte Verbindung ein Polyfluorpolyethersilan der Formel
R*^{f}*Q¹ᵥ[Q²_{w}-[C(R⁴)₂-Si(X)₃₋ₓ(R⁵)x]y]_{z} ist,
worin:
R*^{f}* ein Polyfluorpolyetheranteil ist;
Q¹ eine dreiwertige Verknüpfungsgruppe ist;
jedes Q² eine unabhängig ausgewählte organische zweiwertige oder dreiwertige Verknüpfungsgruppe ist;
jedes R⁴ unabhängig voneinander Wasserstoff oder eine C₁₋₄ -Alkylgruppe ist;
jedes X unabhängig voneinander eine hydrolysierbare oder Hydroxylgruppe ist;
R⁵ eine C₁₋₈ -Alkyl- oder Phenylgruppe ist;
v und w unabhängig voneinander 0 oder 1 sind, x 0 oder 1 oder 2 ist; y 1 oder 2 ist; und z 2, 3 oder 4 ist.

5. Ein Verfahren nach Anspruch 4, worin der Polyfluorpolyetheranteil R*^{f}* perfluorierte wiederkehrende Einheiten umfasst, ausgewählt aus der Gruppe bestehend aus -(CnF₂O)-,- (CF(Z)O)-, -(CF(Z)CnF₂nO)-, -(CnF₂nCF(Z)0)-, - (CF₂CF(Z)O)-, und Kombinationen davon; worin n eine Ganzzahl von 1 bis 6 ist und Z eine Perfluoralkylgruppe, eine sauerstoffhaltige Perfluoralkylgruppe, eine Perfluoralkoxygruppe oder eine sauerstoffsubstituierte Perfluoralkoxygruppe ist, von denen jede linear, verzweigt oder zyklisch sein kann und 1 bis 5 Kohlenstoffatome und bis zu 4 Sauerstoffatome aufweist, wenn sie sauerstoffhaltig oder sauerstoffsubstituiert sind, und worin für sich wiederholende Einheiten, die Z beinhalten, die Anzahl der Kohlenstoffatome in der Folge höchstens 6 beträgt.

6. Ein Verfahren nach einem der vorhergehenden Ansprüche, worin die Oberfläche eine Metalloberfläche ist, insbesondere eine Oberfläche aus einer Aluminiumlegierung, einer Eisenlegierung oder einer Stahllegierung.

7. Ein Verfahren nach einem der vorhergehenden Ansprüche, wobei die medizinische Abgabevorrichtung ein Dosierinhalator oder ein Trockenpulverinhalator ist.

8. Ein Verfahren nach einem der vorhergehenden Ansprüche, worin die Komponente eine Komponente eines Dosierinhalators ist und die Komponente ausgewählt ist aus der Gruppe bestehend aus einem Aktuator, einem Aerosolbehälter, einer Klemmhülse, einem Ventilkörper, einem Ventilschaft und einer Druckfeder.

9. Ein Verfahren nach einem der Ansprüche 1 bis 5, 7 und 8, worin der Abschnitt der Oberfläche eine Polymeroberfläche ist.

10. Ein Verfahren nach Anspruch 9, worin die Komponente zumindest teilweise aus dem Polymer hergestellt ist.

11. Ein Verfahren nach einem der Ansprüche 9 bis 10, worin die zumindest teilweise fluorierte Verbindung Polyfluorpolyethersilan der Formel la ist:
R^{f}[Q¹-[C(R)₂-Si(Y)₃₋ₓ(R^{1a})x]y]z
worin:
R*^{f}* ein monovalenter oder multivalenter Polyfluorpolyetheranteil ist;
Q¹ eine organische zwei- oder dreiwertige Verknüpfungsgruppe ist;
jedes R unabhängig voneinander Wasserstoff oder eine C1-4-Alkylgruppe ist;
jedes Y unabhängig voneinander eine hydrolysierbare Gruppe ist;
R^{1a} eine C1-8-Alkyl- oder Phenylgruppe ist;
x 0 oder 1 oder 2 ist;
y 1 oder 2 ist; und
z 1, 2, 3, oder 4 ist.

12. Ein Verfahren nach Anspruch 11, worin der Polyfluorpolyetheranteil R*^{f}* C₃F₇O(CF(CF₃)CF₂O)ₚCF(CF₃)- ist, worin der Durchschnittswert von p im Bereich zwischen 3 und 50 liegt.

13. Eine beschichtete Komponente für eine medizinische Abgabevorrichtung, umfassend eine Komponente und eine fluorhaltige Beschichtung, worin die fluorhaltige Beschichtung zwei Schichten umfasst, eine erste polyfluorpolyetherhaltige Schicht, umfassend Polyfluorpolyethersilaneinheiten der folgenden Formel Ib:
R*^{f}*[Q¹-[C(R)₂-Si(O-)₃₋ₓ(R^{1a})ₓ]_{y}]_{z} Ib
die mindestens eine kovalente Bindung mit einer zweiten nicht-fluorierten Schicht teilt, umfassend Einheiten der folgenden Formel IIb:
(-O)₃₋ₘ₋ₙ(X)ₙ(R¹)ₘSi-Q-Si(R²)ₖ(X)ₗ(O-)₃₋ₖ₋ₗ IIb
die wiederum mindestens eine kovalente Bindung mit der Komponente teilt; und wobei:
R*^{f}* ein monovalentes oder multivalentes Polyfluorpolyether-Segment ist;
Q¹ eine organische zwei- oder dreiwertige Verknüpfungsgruppe ist;
jedes R unabhängig voneinander Wasserstoff oder eine C1-4-Alkylgruppe ist;
R^{Ia} eine C1-8-Alkyl- oder Phenylgruppe ist;
k, l, m und n unabhängig voneinander 0, 1 oder 2 sind, aber mit der Maßgabe, dass m+n und k+1 höchstens 2 sind;
x 0 oder 1 oder 2 ist;
y 1 oder 2 ist; und
z 1, 2, 3, oder 4 ist;
R¹ und R² unabhängig voneinander ausgewählte einwertige Gruppen sind, X eine hydrolysierbare oder Hydroxygruppe ist, m und k unabhängig voneinander 0, 1 oder 2 sind und Q eine zweiwertige organische Verknüpfungsgruppe ist, umfassend eine substituierte C₂- bis C₁₂-Kohlenwasserstoffkette und eine oder mehrere Amingruppen.

## Revendications

1. Procédé de fabrication d'un composant pour un dispositif de distribution de substance médicinale, le procédé comprenant :
a) la fourniture d'un composant d'un dispositif de distribution de substance médicinale,
b) la fourniture d'une composition d'apprêt comprenant un silane ayant deux groupes silane réactifs ou plus séparés par un groupe de liaison organique,
c) la fourniture d'une composition de revêtement comprenant un composé au moins partiellement fluoré,
d) l'application de la composition d'apprêt sur au moins une partie de la surface du composant,
e) l'application de la composition de revêtement sur la partie de la surface du composant après l'application de la composition d'apprêt.

2. Procédé selon la revendication 1, dans lequel le silane ayant deux groupes silane réactifs ou plus est de formule
X₃₋ₘ(R¹)ₘSi-Q-Si(R²)ₖX₃₋ₖ
dans lequel R¹ et R² sont des groupes univalents choisis indépendamment, X est un groupe hydrolysable ou un groupe hydroxy, m et k sont indépendamment 0, 1, ou 2 et Q est un groupe de liaison organique divalent.

3. Procédé selon la revendication 2, dans lequel Q est de formule - (CH₂)ᵢ-A- (CH₂)ⱼ - dans lequel A est NRⁿ, O, ou S ; i et j sont indépendamment 0, 1, 2, 3, ou 4 et dans lequel Rⁿ est H ou un alkyle C₁ à C₄.

4. Procédé selon l'une quelconque des revendications précédentes, dans lequel le composé partiellement fluoré est un polyfluoropolyéther silane de formule
R^{f}Q¹ᵥ[Q²_{w}-[C(R⁴)₂-Si(X)₃₋ₓ(R⁵)ₓ]_{y}]_{z}
dans lequel :
R*^{f}* est un fragment polyfluoropolyéther ;
Q¹ est un groupe de liaison trivalent ;
chaque Q² est un groupe de liaison organique divalent ou trivalent choisi indépendamment ;
chaque R⁴ est indépendamment de l'hydrogène ou un groupe alkyle C₁₋₄ ;
chaque X est indépendamment un groupe hydrolysable ou hydroxyle ;
R⁵ est un groupe alkyle ou phényle C₁₋₈ ;
v et w sont indépendamment 0 ou 1, x est 0 ou 1 ou 2 ; y est 1 ou 2 ; et z est 2, 3 ou 4.

5. Procédé selon la revendication 4, dans lequel le fragment polyfluoropolyéther R*^{f}* comprend des unités répétitives perfluorées choisies parmi le groupe constitué de -(CₙF₂ₙO)-, -(CF(Z)O)-, -(CF(Z)CₙF₂ₙO)-, -(CₙF₂ₙCF(Z)O)-, -(CF₂CF(Z)O)-, et des combinaisons de ceux-ci ; dans lequel n est un entier de 1 à 6 et Z est un groupe perfluoroalkyle, un groupe perfluoroalkyle contenant de l'oxygène, un groupe perfluoroalcoxy, ou un groupe perfluoroalcoxy à substitution oxygène, chacun d'entre eux pouvant être linéaire, ramifié, ou cyclique, et avoir de 1 à 5 atomes de carbone et jusqu'à 4 atomes d'oxygène lorsqu'ils contiennent de l'oxygène ou qu'ils sont substitués en oxygène et dans lequel pour les unités répétitives incluant Z le nombre d'atomes de carbone en séquence est au maximum de 6.

6. Procédé selon l'une quelconque des revendications précédentes, dans lequel ladite surface est une surface en métal, en particulier une surface d'un alliage d'aluminium, un alliage de fer, ou un alliage d'acier.

7. Procédé selon l'une quelconque des revendications précédentes, dans lequel ledit dispositif de distribution de substance médicinale est un inhalateur doseur ou un inhalateur de poudre sèche.

8. Procédé selon l'une quelconque des revendications précédentes, dans lequel le composant est un composant d'un inhalateur doseur et le composant est choisi dans le groupe constitué d'un actionneur, un récipient d'aérosol, une virole, un corps de soupape, une tige de soupape et un ressort de compression.

9. Procédé selon l'une quelconque des revendications 1 à 5, 7 et 8, dans lequel ladite partie de surface est une surface de polymère.

10. Procédé selon la revendication 9 dans lequel le composant est au moins partiellement composé dudit polymère.

11. Procédé selon l'une quelconque des revendications 9 à 10, dans lequel le composé au moins partiellement fluoré est un polyfluoropolyéther silane de formule la :
R*^{f}*[Q¹-[C(R)₂-Si(Y)₃₋ₓ(R^{1a})ₓ]_{y}]_{z} Ia
dans lequel :
R*^{f}* est un fragment polyfluoropolyéther monovalent ou multivalent ;
Q¹ est un groupe de liaison organique divalent ou trivalent ;
chaque R est indépendamment de l'hydrogène ou un groupe alkyle C1-4 ;
chaque Y est indépendamment un groupe hydrolysable ;
R^{1a} est un groupe alkyle ou phényle C1-8 ;
x est 0 ou 1 ou 2 ;
y est 1 ou 2 ; et
z est 1, 2, 3, ou 4 ;

12. Procédé selon la revendication 11, dans lequel le fragment polyfluoropolyéther R*^{f}* est C₃F₇O(CF(CF₃)CF₂O)ₚCF(CF₃)-, dans lequel la valeur moyenne de p est dans la plage de 3 à 50.

13. Composant revêtu pour un dispositif de distribution de substance médicinale comprenant un composant et un revêtement contenant du fluor, dans lequel le revêtement contenant du fluor comprend deux couches, une première couche contenant du polyfluoropolyéther comprenant des entités de polyfluoropolyéther silane de formule Ib suivante :
R*^{f}*[Q¹-[C(R)₂-Si(O-)₃₋ₓ(R^{1a})ₓ]_{y}]_{z} Ib
qui partage au moins une liaison covalente avec une deuxième couche non fluorée comprenant des entités de la formule IIb suivante :
(-O)₃₋ₘ₋ₙ(X)ₙ(R¹)ₘSi-Q-Si(R²)ₖ(X)ₗ(O-)₃₋ₖ₋ₗ IIb
qui à son tour partage au moins une liaison covalente avec le composant ; et dans lequel :
R*^{f}* est un segment polyfluoropolyéther monovalent ou multivalent ;
Q¹ est un groupe de liaison organique divalent ou trivalent ;
chaque R est indépendamment de l'hydrogène ou un groupe alkyle C1-4 ;
R^{Ia} est un groupe alkyle ou phényle C1-8 ;
k, l, m et n sont indépendamment 0, 1 ou 2, mais à condition que m+n et k+1 soient au plus 2 ;
x est 0 ou 1 ou 2 ;
y est 1 ou 2 ; et
z est 1, 2, 3 ou 4 ;
R¹ et R² sont des groupes univalents choisis indépendamment, X est un groupe hydrolysable ou un groupe hydroxy, m et k sont indépendamment 0, 1, ou 2 et Q est un groupe de liaison organique divalent, comprenant une chaîne hydrocarbyle substituée en C₂ à C₁₂ et un ou plusieurs groupes aminé.
